# EUROPEAN PATENT APPLICATION

(11) **EP 4 737 457 A1**
(43) Date of publication of application: **06.05.2026**
(21) Application number: 24830929.6
(22) Date of filing: 28.06.2024
(51) Int. Cl.: C07D 487/08, A61K 31/4439, A61K 31/513, A61K 31/501, A61P 7/02

(54) **POLYSUBSTITUTED MACROCYCLIC COMPOUND, AND PREPARATION METHOD THEREFOR AND USE THEREOF**

(30) Priority: 30.06.2023 CN 202310799450; 20.07.2023 CN 202310899424
(71) Applicant: Shenzhen Salubris Pharmaceuticals Co., Ltd., Shenzhen, Guangdong 518017 (CN)
(72) Inventor: WU, Junjun, Shenzhen, Guangdong 518017 (CN); LU, Yinsuo, Shenzhen, Guangdong 518017 (CN); XING, Wei, Shenzhen, Guangdong 518017 (CN); XIAO, Ying, Shenzhen, Guangdong 518017 (CN); HUANG, Yiqiang, Shenzhen, Guangdong 518017 (CN); WANG, Liulin, Shenzhen, Guangdong 518017 (CN)
(74) Representative: Graf von Stosch Patentanwaltsgesellschaft mbH
(86) International application number: PCT/CN2024/102166
(87) International publication number: WO 2025/002297

(57) **Abstract**

Provided are a compound as represented by general formula (I), or a racemate thereof, or an isomer thereof, or a pharmaceutically acceptable salt thereof, and a method for treating a plurality of specific diseases or conditions using the same.

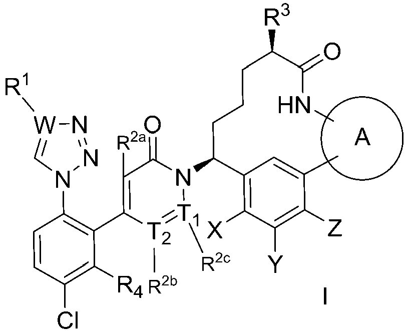

## Description

### TECHNICAL FIELD

The present application belongs to the technical field of chemical pharmaceuticals, and provides a polysubstituted macrocyclic compound and a preparation method therefor and use thereof, as an inhibitor of selective Factor XIa (FXIa). The present application also relates to pharmaceutical compositions comprising these compounds and the use of these compounds in medicaments for treating diseases such as thromboembolism.

### BACKGROUND

Cardiovascular and cerebrovascular diseases such as cerebrovascular diseases, cerebral infarction, myocardial infarction, coronary heart disease, and arteriosclerosis claim nearly 12 million lives worldwide each year, close to one-quarter of the total global death toll, making them the number one enemy of human health. In China, the number of deaths from cardiovascular diseases exceeds 2.6 million annually, with 75% of surviving patients suffering from disability, including over 40% with severe disability. Thrombotic problems caused by cardiovascular and cerebrovascular diseases, diabetes, and their complications have become an urgent issue to be resolved today.

The human blood coagulation process consists of the intrinsic pathway, the extrinsic pathway, and the common pathway (Annu. Rev. Med. 2011.62:41-57). It is a cascade reaction where the process is continuously strengthened and amplified through the sequential activation of multiple zymogens. The coagulation cascade is initiated by the intrinsic pathway (also known as the contact activation pathway) and the extrinsic pathway (also known as the tissue factor pathway) to generate FXa, which then proceeds through the common pathway to generate thrombin (FIIa), ultimately forming fibrin.

The intrinsic pathway refers to the process where Factor XII is activated to form the XIa-VIIIa-Ca²⁺-PL complex and activates Factor X, while the extrinsic coagulation pathway is the process where tissue factor (TF) is released to form the TF-FVIIa-Ca²⁺ complex and activates Factor X. The common pathway refers to the process after Factor Xa is formed, where the two pathways merge, activating prothrombin and ultimately generating fibrin. FXI is essential for maintaining the intrinsic pathway and plays a key role in the amplification process of the coagulation cascade. In the coagulation cascade, thrombin can feedback-activate FXI, and activated FXI (FXIa) in turn promotes the massive production of thrombin, thereby amplifying the coagulation cascade. Therefore, antagonists of FXI are widely developed for the treatment of various thromboses.

Traditional anticoagulant drugs, such as warfarin, heparin, low molecular weight heparin (LMWH), and recently marketed new drugs, such as FXa inhibitors (rivaroxaban, apixaban, etc.) and thrombin inhibitors (dabigatran etexilate, hirudin, etc.), have good effects on reducing thrombus formation and occupy a large cardiovascular and cerebrovascular market due to their significant effectiveness. However, their side effects are becoming increasingly significant, among which the "bleeding risk" is one of the most severe and primary concerns (N Engl J Med 1991;325:153-8, Blood.2003;101:4783-4788).

Studies have found that in thrombosis models, inhibiting the FXIa factor can effectively inhibit thrombus formation, but in more severe thrombotic conditions, the role of FXIa is minimal (Blood.2010;116(19):3981-3989). Clinical statistics show that increasing the amount of FXIa increases the prevalence of VTE (Blood 2009;114:2878-2883), while those with severe FXIa deficiency have a reduced risk of DVT (Thromb Haemost 2011;105:269-273).

FXIa, as an emerging target for inhibiting thrombosis, has been the subject of several patent applications disclosing compounds with FXIa inhibitory activity, including WO9630396, WO9941276, WO2013093484, WO2004002405, WO2013056060, WO2017005725, WO2017/023992, WO2018041122, etc.

### SUMMARY

The present application provides a series of macrocyclic derivatives, a preparation method therefor, and use thereof in medicine.

Specifically, in a first aspect, the present application provides a compound represented by general formula (I), or an isomer thereof, or a racemate thereof, or a pharmaceutically acceptable salt thereof:

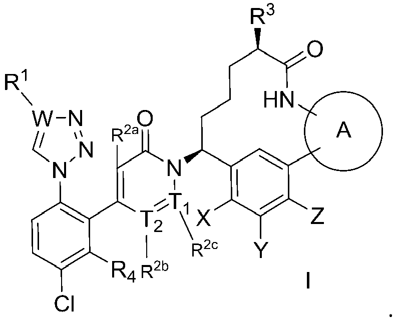

In a second aspect, the present application further provides a pharmaceutical composition comprising a therapeutically effective amount of the compound or a pharmaceutically acceptable salt thereof according to any one of the preceding, and a pharmaceutically acceptable carrier.

In a third aspect, the present application also provides a medical use of a therapeutically effective amount of the aforementioned compound or a pharmaceutically acceptable salt thereof, specifically, use in the manufacture of a medicament for treating a condition as an inhibitor of selective Factor XIa (FXIa), and use in the manufacture of a medicament for treating a disease such as thromboembolism.

Specifically, the present application is achieved through the following technical solution:
A compound represented by general formula (I), or an isomer thereof, or a racemate thereof, or a pharmaceutically acceptable salt thereof, 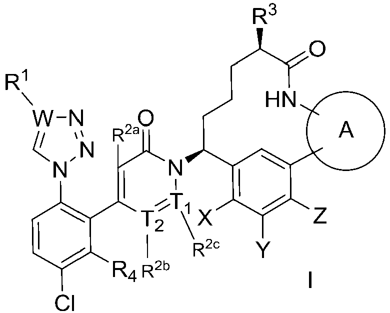
wherein X is selected from the group consisting of hydrogen, halogen, and cyano; Y is selected from the group consisting of hydrogen, halogen, and cyano; Z is selected from the group consisting of hydrogen, halogen, and cyano; and at least two of X, Y, and Z are not hydrogen;
W is selected from the group consisting of C and N, wherein when W is N, R¹ is absent;
T₁ and T₂ are selected from the group consisting of C and N, wherein when T₁ is N, R^{2c} is absent, when T₂ is N, R^{2b} is absent, and T₁ and T₂ cannot both be N;
ring A is selected from the group consisting of a substituted or unsubstituted benzene ring and a substituted or unsubstituted pyrazole ring, wherein a substituent is selected from the group consisting of halogen, cyano, alkyl, and haloalkyl;
R¹ is selected from the group consisting of halogen, haloalkyl, and cyano;
R^{2a}, R^{2b}, and R^{2c} are each independently selected from the group consisting of hydrogen, halogen, and alkoxy;
R³ is selected from the group consisting of alkyl, haloalkyl, alkoxy, -(CH₂)ₙ-cycloalkyl, and -(CH₂)ₙ-heterocycloalkyl, wherein n is 0 or 1; and
R⁴ is selected from the group consisting of hydrogen and halogen.

As a preferred technical solution of the present application, the compound is selected from the group consisting of a compound represented by formula (Ia) or formula (Ib), or an isomer thereof, or a racemate thereof, or a pharmaceutically acceptable salt thereof: 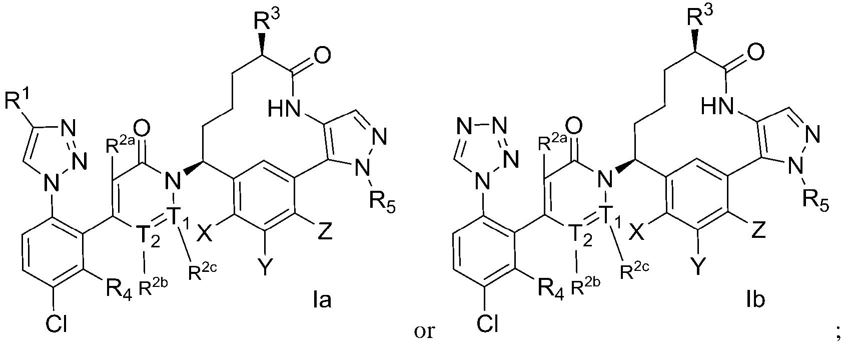
wherein X is selected from the group consisting of hydrogen, halogen, and cyano; Y is selected from the group consisting of hydrogen, halogen, and cyano; Z is selected from the group consisting of hydrogen, halogen, and cyano; and at least two of X, Y, and Z are not hydrogen;
T₁ and T₂ are selected from the group consisting of C and N, wherein when T₁ is N, R^{2c} is absent, when T₂ is N, R^{2b} is absent, and T₁ and T₂ cannot both be N;
R¹ is selected from the group consisting of halogen, haloalkyl, and cyano;
R^{2a}, R^{2b}, and R^{2c} are each independently selected from the group consisting of hydrogen, halogen, and alkoxy;
R³ is selected from the group consisting of alkyl, haloalkyl, alkoxy, -(CH₂)ₙ-cycloalkyl, -(CH₂)ₙ-heterocycloalkyl, wherein n is 0 or 1;
R⁴ is selected from the group consisting of hydrogen and halogen; and
R⁵ is selected from the group consisting of alkyl and haloalkyl.

As a preferred technical solution of the present application, the alkyl is selected from the group consisting of C₁₋₆ alkyl, wherein the C₁₋₆ alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present application, the alkoxy is selected from the group consisting of C₁₋₆ alkoxy, wherein the C₁₋₆ alkoxy is selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexyloxy, isohexyloxy, sec-hexyloxy, tert-hexyloxy, neohexyloxy, 2-methylpentyloxy, 1,2-dimethylbutoxy, and 1-ethylbutoxy.

As a preferred technical solution of the present application, the halogen is selected from the group consisting of fluorine, chlorine, bromine, and iodine.

As a preferred technical solution of the present application, the haloalkyl means one or more hydrogens on the alkyl are replaced by halogen, wherein the halogen is selected from the group consisting of fluorine, chlorine, bromine, and iodine.

As a preferred technical solution of the present application, the cycloalkyl is selected from the group consisting of C₃₋₆ cycloalkyl, wherein the C₃₋₆ cycloalkyl is selected from the group consisting of cyclopropyl, cyclobutyl, cyclopentyl, and cyclohexyl; the heterocycloalkyl means one or more carbon atoms in the cycloalkyl group are replaced by a heteroatom, wherein the heteroatom is selected from the group consisting of nitrogen, oxygen, and sulfur, and a number of the heteroatom is one or more.

As a preferred technical solution of the present application,
W is selected from the group consisting of C and N, wherein when W is N, R¹ is absent;
X is selected from the group consisting of hydrogen and fluorine; Y is selected from the group consisting of hydrogen and fluorine; Z is selected from the group consisting of hydrogen and fluorine; and at least two of X, Y and Z are not hydrogen;
T₁ and T₂ are selected from the group consisting of C and N, wherein when T₁ is N, R^{2c} is absent, when T₂ is N, R^{2b} is absent, and T₁ and T₂ cannot both be N;
R¹ is selected from the group consisting of chlorine, trifluoromethyl, and difluoromethyl; R^{2a}, R^{2b}, and R^{2c} are each independently selected from the group consisting of hydrogen, methoxy, fluorine, and chlorine; R³ is methyl; R⁴ is selected from the group consisting of hydrogen and fluorine; and R⁵ is selected from the group consisting of methyl and difluoromethyl.

As a preferred technical solution of the present application, one or more hydrogen atoms of the compound are replaced by isotope deuterium (²H).

As a preferred technical solution of the present application, the compound is selected from the group consisting of:

| No. | Compound Structure | No. | Compound Structure |
|---|---|---|---|
| 1 | 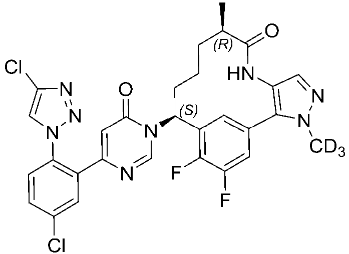 | 2 | 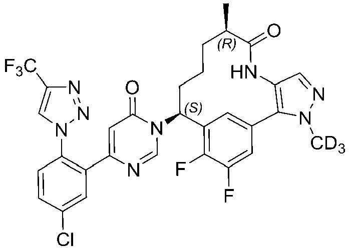 |
| 3 | 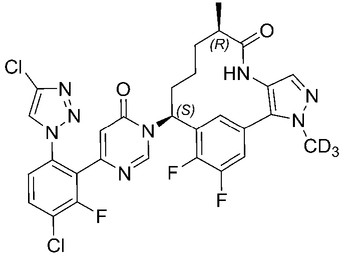 | 4 | 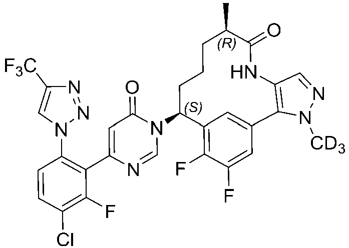 |
| 5 | 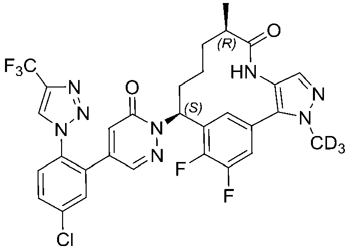 | 6 | 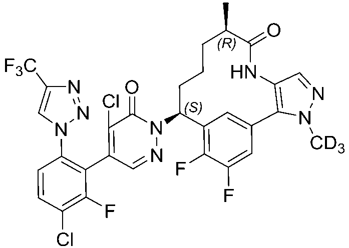 |
| 7 | 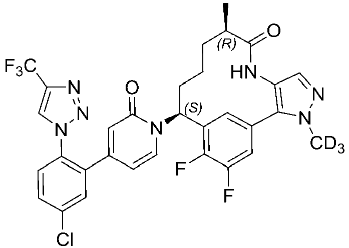 | 8 | 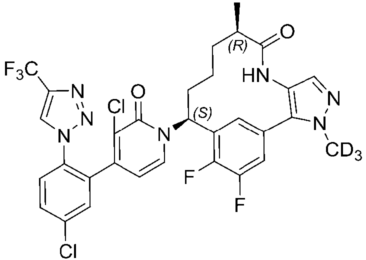 |
| 9 | 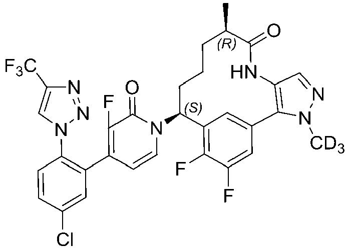 | 10 | 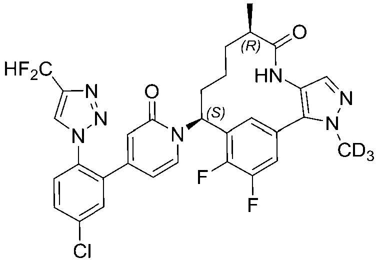 |
| 11 | 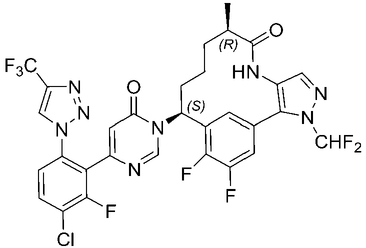 | 12 | 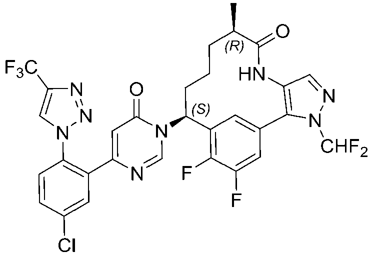 |
| 13 | 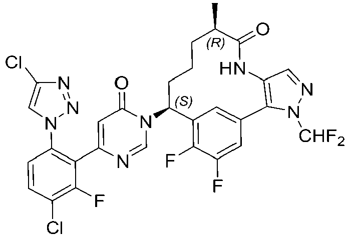 | 14 | 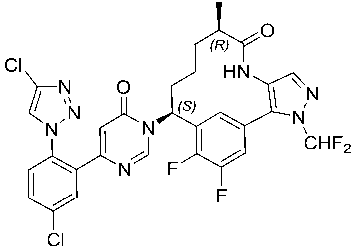 |
| 15 | 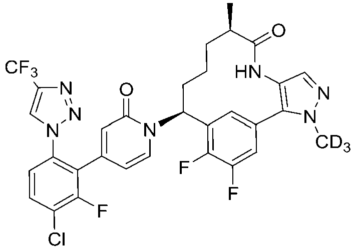 | 16 | 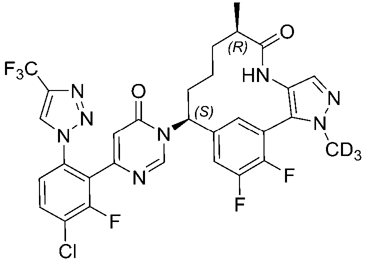 |
| 17 | 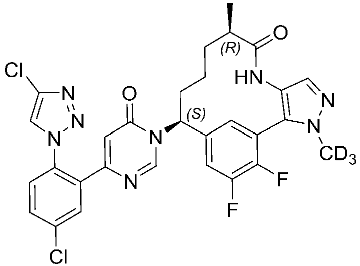 | 18 | 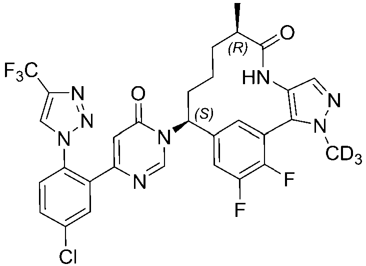 |
| 19 | 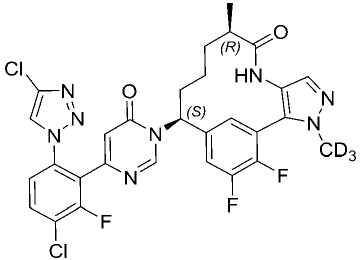 | 20 | 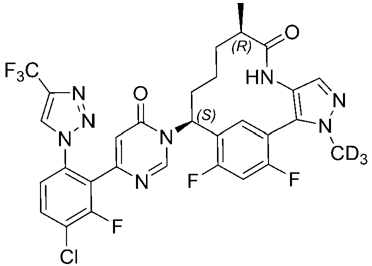 |
| 21 | 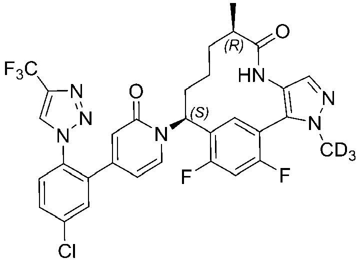 | 22 | 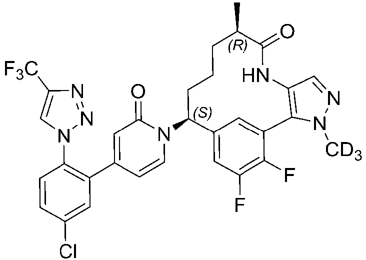 |
| 23 | 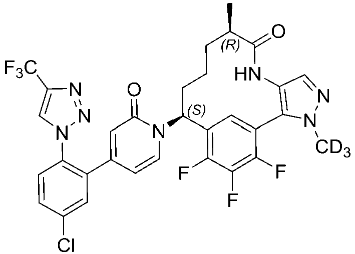 | | |

As a preferred technical solution of the present application, a pharmaceutically acceptable salt of the compound is provided, wherein the pharmaceutically acceptable salt of the compound refers to the compound, or the isomer thereof, or the racemate thereof, prepared with a pharmaceutically acceptable acid or base.

As a preferred technical solution of the present application, a pharmaceutical composition is provided, comprising a therapeutically effective amount of the aforementioned compound, or the isomer thereof, or the racemate thereof, or the pharmaceutically acceptable salt thereof, and a pharmaceutically acceptable carrier.

As a preferred technical solution of the present application, a medical use of the compound, or the isomer thereof, or the racemate thereof, or the pharmaceutically acceptable salt thereof is provided, specifically, use in the manufacture of a medicament for treating an FXIa-related disease, preferably use in the manufacture of a medicament for treating a thrombus-related disease.

For clarity, general terms used in the description of the compounds are defined herein.

Unless otherwise stated, the following terms and phrases used herein are intended to have the following meanings. A specific term or phrase that is not specifically defined should not be considered indefinite or unclear, but should be understood according to its ordinary meaning. When a trade name appears herein, it is intended to refer to the corresponding commercial product or its active ingredient. The term "pharmaceutically acceptable" as used here refers to those compounds, materials, compositions, and/or dosage forms that are within the scope of sound medical judgment, suitable for use in contact with the tissues of humans and animals without excessive toxicity, irritation, allergic reactions, or other problems or complications, commensurate with a reasonable benefit/risk ratio.

The term "pharmaceutically acceptable salt" refers to a salt of the compound of the present application, prepared from the compound discovered in the present application having specific substituents with a pharmaceutically acceptable acid or base.

In addition to salt forms, the compounds provided by the present application can also exist in prodrug forms. Prodrugs of the compounds described herein readily undergo chemical changes under physiological conditions to convert into the compounds of the present application. Additionally, prodrugs can be converted to the compounds of the present application by chemical or biochemical methods in an in vivo environment.

Certain compounds of the present application may exist in unsolvated forms or solvated forms, including hydrated forms. Generally, solvated forms are equivalent to unsolvated forms and are all encompassed within the scope of the present application.

The compounds of the present application may exist in specific geometric or stereoisomeric forms. The present application contemplates all such compounds, including *cis-* and *trans*-isomers, (-)- and (+)-enantiomers, (*R*)- and (*S*)-enantiomers, diastereomers, (*D*)-isomers, (*L*)-isomers, and racemic mixtures thereof and other mixtures, such as enantiomerically or diastereomerically enriched mixtures, all of which are encompassed within the scope of the present application. Additional asymmetric carbon atoms may be present in substituents such as alkyl groups. All such isomers and mixtures thereof are included within the scope of the present application.

Optically active (R)- and (S)-isomers, as well as D and L isomers, can be prepared by chiral synthesis or chiral reagents or other conventional techniques. If one enantiomer of a compound of the present application is desired, it may be prepared by asymmetric synthesis or by derivation with a chiral auxiliary, where the resulting diastereomeric mixture is separated and the auxiliary group is cleaved to provide the pure desired enantiomer. Alternatively, when the molecule contains a basic functional group (such as amino) or an acidic functional group (such as carboxyl), diastereomeric salts are formed with an appropriate optically active acid or base, followed by resolution of the diastereomers by conventional methods known in the art, and then recovery of the pure enantiomer. Furthermore, the separation of enantiomers and diastereomers is often accomplished using chromatography employing a chiral stationary phase, optionally combined with chemical derivatization (e.g., generation of carbamates from amines).

Atoms of the compound molecules of the present application may be isotopes. Isotopic derivatization often leads to effects such as prolonged half-life, reduced clearance, metabolic stability, and improved in vivo activity. Furthermore, one embodiment includes where at least one atom is replaced by an atom having the same atomic number (proton number) and a different mass number (sum of protons and neutrons). Examples of isotopes included in the compounds of the present application include hydrogen atoms, carbon atoms, nitrogen atoms, oxygen atoms, phosphorus atoms, sulfur atoms, fluorine atoms, and chlorine atoms, which include ²H, ³H, ¹³C, ¹⁴C, ¹⁵N, ¹⁷O, ¹³O, ³¹P, ³²P, ³⁵S, ¹⁸F, ³⁶Cl, respectively. In particular, radioactive isotopes that emit radiation upon their decay, such as ³H or ¹⁴C, can be used for drug formulation or anatomical testing of compounds in vivo. Stable isotopes neither decay nor change in amount and are non-radioactive, thus they can be used safely. When atoms constituting the compound molecules of the present application are isotopes, the isotopes can be incorporated according to general methods by replacing the reagents used in the synthesis with reagents containing the corresponding isotopes.

The compounds of the present application may comprise a non-natural proportion of an atomic isotope at one or more atoms that constitute such compounds. For example, the compounds can be labeled with radioactive isotopes, such as deuterium (²H), iodine-125 (¹²⁵I), or carbon-14 (¹⁴C). All isotopic variations of the compounds of the present application, whether radioactive or not, are encompassed within the scope of the present application.

Further, in the compounds of the present application, one or more hydrogen atoms are replaced by isotope deuterium (²H). Deuterated compounds of the present application exhibit effects such as prolonged half-life, reduced clearance, metabolic stability, and improved in vivo activity.

The preparation method for the isotopic derivatives generally includes: phase transfer catalysis methods. For example, a preferred deuteration method employs a phase transfer catalyst (e.g., a tetraalkylammonium salt, NBu₄HSO₄). Using a phase transfer catalyst to exchange the methylene protons of a diphenylmethane compound results in higher deuterium incorporation compared to reduction using deuterated silane (e.g., triethyl deuterated silane) in the presence of an acid (e.g., methanesulfonic acid) or using a Lewis acid such as aluminum trichloride with sodium deuterated borohydride.

The term "pharmaceutically acceptable carrier" refers to any formulation carrier or medium capable of delivering an effective amount of the active substance of the present application, which does not interfere with the biological activity of the active substance and has no toxic or side effects on the host or patient. Representative carriers include water, oil, vegetable and mineral substances, cream bases, lotion bases, ointment bases, etc. These bases include suspending agents, viscosity enhancers, transdermal enhancers, etc. Their formulations are well known to those skilled in the art of cosmetics or topical pharmaceuticals. For further information on carriers, reference can be made to Remington: The Science and Practice of Pharmacy, 21st Ed., Lippincott, Williams & Wilkins (2005), the content of which is incorporated herein by reference.

The term "excipient" generally refers to the carrier, diluent, and/or medium required to formulate an effective pharmaceutical composition.

With respect to a drug or pharmacologically active agent, the terms "effective amount" or "therapeutically effective amount" refer to a sufficient amount of the drug or agent that is non-toxic but achieves the desired effect. For the oral dosage forms in the present application, the "effective amount" of one active substance in the composition refers to the amount required to achieve the desired effect when used in combination with another active substance in the composition. The determination of an effective amount varies from person to person, depending on the age and general condition of the recipient, as well as the specific active substance. A suitable effective amount in individual cases can be determined by those skilled in the art through routine experimentation.

The terms "active ingredient," "therapeutic agent," "active substance," or "active agent" refer to a chemical entity that is effective in treating the targeted disorder, disease, or condition.

"Optional" or "optionally" means that the subsequently described event or circumstance may but need not occur, and that the description includes instances where the event or circumstance occurs and instances where it does not.

The compounds of the present application can be prepared by a variety of synthetic methods well known to those skilled in the art, including the specific embodiments listed below, embodiments formed by their combination with other chemical synthesis methods, and equivalent alternatives well known to those skilled in the art. Preferred embodiments include, but are not limited to, the examples of the present application.

### DETAILED DESCRIPTION

The present application is described in further detail below with reference to examples, but the embodiments of the present application are not limited thereto.

### Example 1

### Synthesis of (3S,7R)-3-(4-(5-chloro-2-(4-chloro-1H-1,2,3-triazol-1-yl)phenyl)-6-oxopyrimidin-1(6H)-yl)-2⁴,2⁵-difluoro-7-methyl-1¹-(methyl-d₃)-1¹H-9-aza-1(5,4)-pyrazola-2( 1,3)-benzenacyclononaphan-8-one (1)

The specific synthetic route is as follows:

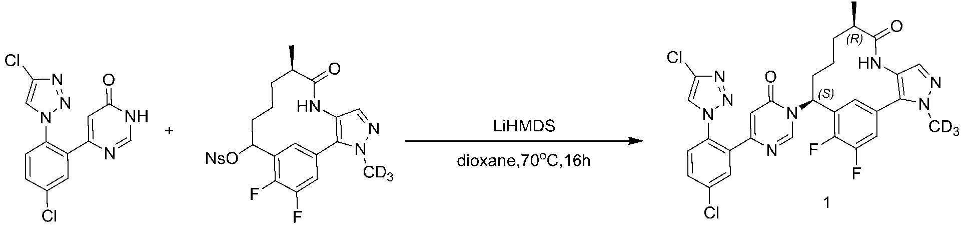

6-(5-Chloro-2-(4-chloro-1*H*-1,2,3-triazol-1-yl)phenyl)pyrimidin-4(3*H*)-one (53.0 mg, 0.173 mmol) was dissolved in dry 1,4-dioxane (6 mL). Lithium bis(trimethylsilyl)amide (1 M solution in tetrahydrofuran, 0.173 mL, 0.173 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (90.3 mg, 0.173 mmol) was added, and the reaction was carried out at 70 °C for 16 hours.

After monitoring indicated completion of the reaction, the mixture was cooled to room temperature. Saturated aqueous ammonium chloride solution (25 mL) and ethyl acetate (30 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 17.9 mg of (3S,7R)-3-(4-(5-chloro-2-(4-chloro-1*H*-1,2,3-triazol-1-yl)phenyl)-6-oxopyrimidin-1(6*H*)-yl)-2⁴,2⁵-difluoro-7-methyl-1¹-(methyl-*d*₃)-1¹*H*-9-aza-1(5,4) -pyrazola-2(1,3)-benzenacyclononaphan-8-one as a white solid (yield: 16.5%). LC-MS: RT = 2.00 min, [M+H]⁺ = 627.99. ¹H NMR (400 MHz, DMSO-*d*₆) δ 9.23 (s, 1H), 8.71 (s, 1H), 8.27 (s, 1H), 7.91 (d, *J =* 2.3 Hz, 1H), 7.86-7.80 (m, 2H), 7.75 (d, *J* = 8.5 Hz, 1H), 7.54 (d, *J =* 6.0 Hz, 1H), 7.45 (s, 1H), 6.46 (s, 1H), 5.63 (d, *J* = 12.3 Hz, 1H), 2.46 - 2.36 (m, 2H), 1.98 - 1.91 (m, 1H), 1.79 - 1.71 (m, 1H), 1.51 - 1.37 (m, 2H), 1.11 - 1.05 (m, 1H), 1.02 (d, *J =* 6.7 Hz, 3H).

### Example 2

### Synthesis of (3R,7R)-3-(4-(5-chloro-2-(4-(trifluoromethyl)-1H-1,2,3-triazol-1-yl)phenyl) -6-oxopyrimidin-1(6H)-yl)-2⁴2⁵-difluoro-7-methyl-1¹-(methyl-d₃)-1¹H-9-aza-1(5,4)-pyrazola -2(1,3)-benzenacyclononaphan-8-one (2)

The specific synthetic route is as follows:

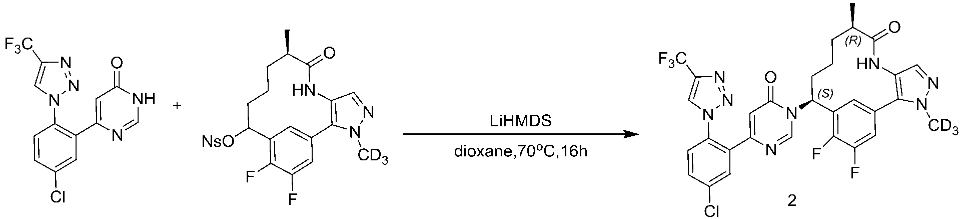

6-(5-Chloro-2-(4-(trifluoromethyl)-1*H*-1,2,3-triazol-1-yl)phenyl)pyrimidin-4(3*H*)-one (80.0 mg, 0.234 mmol) was dissolved in dry 1,4-dioxane (8 mL). Lithium bis(trimethylsilyl)amide (1 M solution in tetrahydrofuran, 0.234 mL, 0.234 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (122 mg, 0.234 mmol) was added, and the reaction was carried out at 70 °C for 16 hours.

After monitoring indicated completion of the reaction, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 33.2 mg of (3*R*,7*R*)-3-(4-(5-chloro-2-(4-(trifluoromethyl)-1*H-*1,2,3-triazol-1-yl)phenyl)-6-oxopyrimidin-1(6*H*)-yl)-2⁴,2⁵-difluoro-7-methyl-1¹-(methyl-*d*₃)-1¹*H* -9-aza-1(5,4)-pyrazola-2(1,3)-benzenacyclononaphan-8-one as a white solid (yield: 21.4%). LC-MS: RT = 1.98 min, [M+H]⁺ = 662.08. ¹H NMR (400 MHz, DMSO-*d*₆) δ 9.23 (s, 1H), 9.15 (s, 1H), 8.20 (s, 1H), 7.94 (d, *J =* 2.0 Hz, 1H), 7.87 - 7.80 (m, 3H), 7.53 (d, *J =* 5.9 Hz, 1H), 7.45 (s, 1H), 6.57 (s, 1H), 5.62 (d, *J* = 12.2 Hz, 1H), 2.46-2.36 (m, 2H), 1.92 7-1.87(m, 1H), 1.78-1.69(m, 1H), 1.53-1.35 (m, 2H), 1.09-1.04 (m, 1H), 1.02(d, *J =* 6.7 Hz, 3H).

### Example 3

### Synthesis of (3S,7R)-3-(4-(3-chloro-6-(4-chloro-1H-1,2,3-triazol-1-yl)-2-fluorophenyl)-6-oxopyrimidin-1(6H)-yl)-2⁴,2⁵-difluoro-7-methyl-1¹-(methyl-d₃)-1¹H-9-aza-1(5,4)-pyrazola-2(1,3)-benzenacyclononaphan-8-one (3)

The specific synthetic route is as follows:

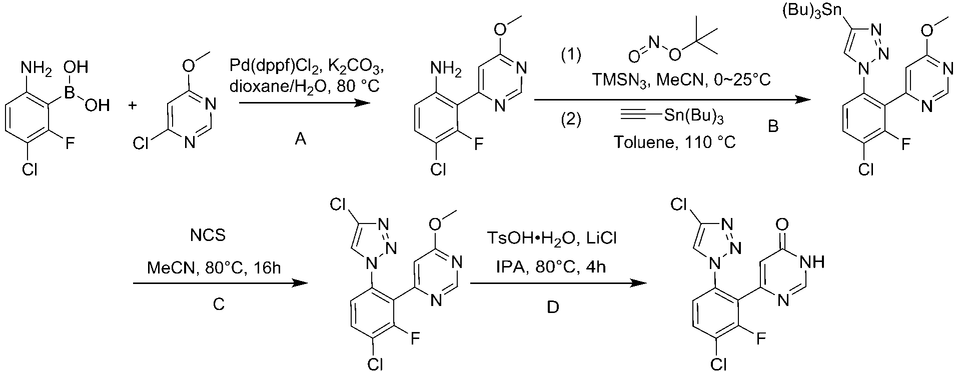

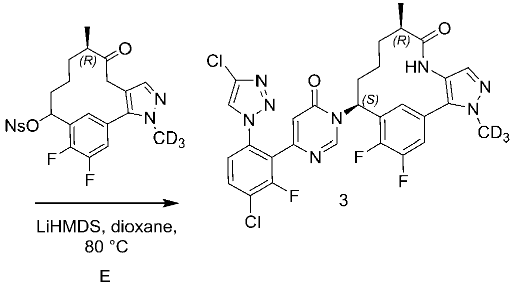

### Step A: Synthesis of 4-chloro-3-fluoro-2-(6-methoxypyrimidin-4-yl)aniline

(6-Amino-3-chloro-2-fluorophenyl)boronic acid (1.60 g, 8.47 mmol), 4-chloro-6-methoxypyrimidine (1.80 g, 12.70 mmol), and potassium carbonate (2.30 g, 16.91 mmol) were dissolved in 1,4-dioxane (40 mL) and water (8 mL). A palladium catalyst (619 mg, 0.850 mmol) was added, and the mixture was stirred at 80 °C for 1 hour under nitrogen protection. After completion of the reaction, water and ethyl acetate were added for extraction. The organic phase was dried over anhydrous sodium sulfate and concentrated under reduced pressure. The residue was purified by silica gel column chromatography (eluent: ethyl acetate/n-hexane = 1/2) to afford 1.20 g of 4-chloro-3-fluoro-2-(6-methoxypyrimidin-4-yl)aniline as a white solid (yield: 56.1%). LCMS: RT = 2.29 min, [M+H]⁺ = 254.08.

### Step B: Synthesis of 4-(3-chloro-6-(4-(tributylstannyl)-1H-1,2,3-triazol-1-yl)-2-fluoro phenyl)-6-methoxypyrimidine

4-Chloro-3-fluoro-2-(6-methoxypyrimidin-4-yl)aniline (1.20 g, 4.74 mmol) was dissolved in acetonitrile (50 mL). tert-Butyl nitrite (1.5 mL) was added at 0 °C, followed by dropwise addition of trimethylsilyl azide (1.5 mL). The reaction mixture was stirred at room temperature for 1 hour.

After TLC monitoring indicated completion of the reaction, water and ethyl acetate were added for extraction. The organic phase was dried over anhydrous sodium sulfate and concentrated under reduced pressure. The residue was dissolved in toluene (50 mL), and tributylstannylacetylene (1.5 mL) was added. The reaction mixture was stirred at 110 °C for 6 hours.

After TLC monitoring indicated completion of the reaction, the mixture was concentrated under reduced pressure. The crude product was purified by silica gel column chromatography (eluent: ethyl acetate/n-hexane = 1/6) to afford 1.50 g of 4-(3-chloro-6-(4-(tributylstannyl)-1*H-*1,2,3-triazol-1-yl)-2-fluorophenyl)-6-methoxypyrimidine as a pale yellow solid (yield: 54.1%). LCMS: RT = 2.23 min.

### Step C: Synthesis of 4-(3-chloro-6-(4-chloro-1H-1,2,3-triazol-1-yl)-2-fluorophenyl)-6-methoxypyrimidine

4-(3-Chloro-6-(4-(tributylstannyl)-1*H*-1,2,3-triazol-1-yl)-2-fluorophenyl)-6-methoxypyrimi dine (1.50 g, 2.52 mmol) was dissolved in acetonitrile (15 mL). N-Chlorosuccinimide (490 mg, 3.66 mmol) was added, and the reaction mixture was stirred at 90 °C for 16 hours.

After TLC monitoring indicated completion of the reaction, water and ethyl acetate were added for extraction. The organic phase was dried over anhydrous sodium sulfate and concentrated under reduced pressure. The crude product was purified by silica gel column chromatography (eluent: ethyl acetate/n-hexane = 1/5) to afford 800 mg of 4-(3-chloro-6-(4-chloro-1*H*-1,2,3-triazol-1-yl)-2-fluorophenyl)-6-methoxypyrimidine as a pale yellow solid (yield: 93.1%). LCMS: RT = 2.08 min, [M+H]⁺ = 340.10.

### Step D: Synthesis of 4-(3-chloro-6-(4-chloro-1H-1,2,3-triazol-1-yl)-2-fluorophenyl) pyrimidin-2(1H)-one

4-(3-Chloro-6-(4-chloro-1*H*-1,2,3-triazol-1-yl)-2-fluorophenyl)-6-methoxypyrimidine (800 mg, 2.35 mmol) was dissolved in isopropanol (15 mL). Lithium chloride (1.44 g, 33.5 mmol) and p-toluenesulfonic acid monohydrate (2.73 g, 14.3 mmol) were added. The reaction mixture was stirred at 80 °C for 4 hours. After TLC monitoring indicated completion of the reaction, water and ethyl acetate were added for extraction. The organic phase was dried over anhydrous sodium sulfate and concentrated under reduced pressure. The residue was triturated (ethyl acetate/n-hexane = 1/10) to afford 730 mg of 4-(3-chloro-6-(4-chloro-1*H*-1,2,3-triazol-1-yl)-2-fluorophenyl)pyrimidin-2(1*H*)-one as a white solid (yield: 95.1%). LCMS: RT = 1.93 min, [M+H]⁺ = 326.01.

### Step E: Synthesis of (3S,7R)-3-(4-(3-chloro-6-(4-chloro-1H-1,2,3-triazol-1-yl)-2-fluorophenyl)-6-oxopyrimidin-1(6H)-yl)-2⁴2⁵-difluoro-7-methyl-1¹-(methyl-d₃)-1¹H-9-aza-1 (5,4)-pyrazola-2(1,3)-benzenacyclononaphan-8-one

4-(3-Chloro-6-(4-chloro-1*H*-1,2,3-triazol-1-yl)-2-fluorophenyl)pyrimidin-2(1*H*)-one (100 mg, 0.310 mmol) was dissolved in dry 1,4-dioxane (10 mL). Lithium bis(trimethylsilyl)amide (1 M solution in tetrahydrofuran, 0.330 mL, 0.330 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphan-3-yl 4-nitrobenzenesulfonate (100 mg, 0.190 mmol) was added, and the reaction mixture was stirred at 80 °C for 24 hours.

After TLC monitoring indicated completion of the reaction, the mixture was cooled to room temperature. Saturated aqueous ammonium chloride solution (25 mL) and ethyl acetate (30 mL) were added. The mixture was separated, and the organic phase was washed with saturated brine, dried over anhydrous sodium sulfate, and concentrated under reduced pressure. The residue was purified by silica gel column chromatography (eluent: ethyl acetate/petroleum ether = 10/1), followed by purification using preparative high-performance liquid chromatography to afford 47.0 mg of (3*S*,7*R*)-3-(4-(3-chloro-6-(4-chloro-1*H*-1,2,3-triazol-1-yl)-2-fluorophenyl)-6-oxopyrimidin-1(6*H*)-yl)-2⁴,2⁵-difluoro-7-methyl-1¹-(methyl-*d*₃)-1¹*H*-9-aza-1(5,4)-pyrazola-2(1,3 )-benzenacyclononaphan-8-one as a white solid (yield: 39.0). LC-MS: RT = 1.93 min, [M+H]⁺ =646.01. ¹H NMR (400 MHz, DMSO-*d*₆) δ 9.24 (s, 1H), 8.68 (s, 1H), 8.33 (s, 1H), 8.02 (t, *J =* 8.2 Hz, 1H), 7.89-7.75 (m, 1H), 7.68 (dd, *J =* 8.7, 1.4 Hz, 1H), 7.54 (d, *J =* 6.0 Hz, 1H), 7.45 (s, 1H), 6.66 (s, 1H), 5.69-5.60 (m, 1H), 2.47-2.37 (m, 2H), 2.03-1.90 (m, 1H), 1.80-1.70 (m, 1H), 1.50-1.37 (m, 2H), 1.13-0.97 (m, 4H).

### Example 4

### Synthesis of (3S,7R)-3-(4-(3-chloro-2-fluoro-6-(4-(trifluoromethyl)-1H-1,2,3-triazol-1-yl)phenyl)-6-oxopyrimidin-1(6H)-yl)-2⁴,2⁵-difluoro-7-methyl-1¹-(methyl-d₃)-1¹H-9-aza-1(5 ,4)-pyrazola-2(1,3)-benzenacyclononaphan-8-one (4)

The specific synthetic route is as follows:

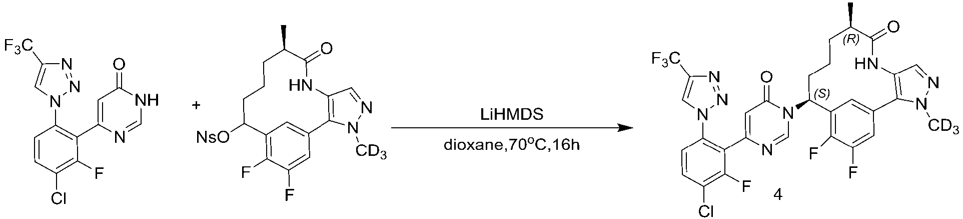

6-(3-Chloro-2-fluoro-6-(4-(trifluoromethyl)-1*H*-1,2,3-triazol-1-yl)phenyl)pyrimidin-4(3*H*)-one (80.0 mg, 0.222 mmol) was dissolved in dry 1,4-dioxane (8 mL). Lithium bis(trimethylsilyl)amide (1 M solution in tetrahydrofuran, 0.222 mL, 0.222 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (116 mg, 0.222 mmol) was added, and the reaction was carried out at 70 °C for 16 hours.

After monitoring indicated completion of the reaction, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 27.3 mg of (3*S*,7*R*)-3-(4-(3-chloro-2-fluoro-6-(4-(trifluoromethyl)-1*H*-1,2,3-triazol-1-yl)phenyl)-6-oxopyrim idin-1(6H)-yl)-2⁴,2⁵-difluoro-7-methyl-1¹-(methyl-*d*₃)-1¹*H*-9-aza-1(5,4)-pyrazola-2(1,3)-benzena cyclononaphan-8-one as a white solid (yield: 18.1%). LC-MS: RT = 1.97 min, [M+H]⁺ = 680.03.

¹H NMR (400 MHz, DMSO-*d*₆) δ 9.23 (s, 1H), 9.15 (s, 1H), 8.26 (s, 1H), 8.05 (dd, *J* = 8.7, 7.7 Hz, 1H), 7.87 - 7.80 (m, 1H), 7.77 (dd, *J* = 8.8, 1.4 Hz, 1H), 7.52 (d, *J* = 5.9 Hz, 1H), 7.45 (s, 1H), 6.71 (s, 1H), 5.64 (d, *J* = 12.2 Hz, 1H), 2.46 - 2.36 (m, 2H), 2.01 - 1.90 (m, 1H), 1.81 - 1.69 (m, 1H), 1.54 - 1.37 (m, 2H), 1.11 - 1.04 (m, 1H), 1.01 (d, *J* = 6.7 Hz, 3H).

### Example 5

### Synthesis of (3S,7R)-3-(4-(5-chloro-2-(4-(trifluoromethyl)-1H-1,2,3-triazol-1-yl)phenyl) -6-oxopyridazin-1(6H)-yl)-2⁴,2⁵-difluoro-7-methyl-1¹-(methyl-d₃)-1¹H-9-aza-1(5,4)-pyrazola -2(1,3)-benzenacyclononaphan-8-one (5)

The specific synthetic route is as follows:

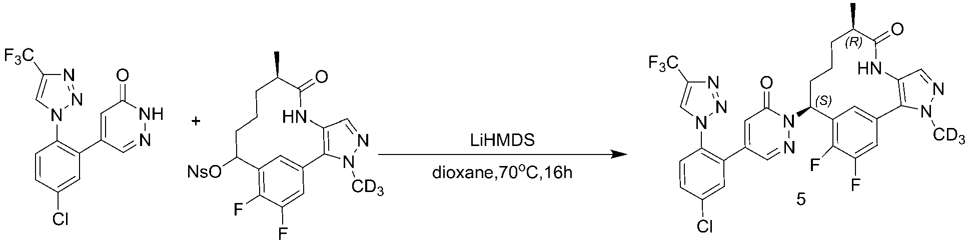

5-(5-Chloro-2-(4-(trifluoromethyl)-1*H*-1,2,3-triazol-1-yl)phenyl)pyridazin-3(2*H*)-one (80.0 mg, 0.234 mmol) was dissolved in dry 1,4-dioxane (8 mL). Lithium bis(trimethylsilyl)amide (1 M solution in tetrahydrofuran, 0.234 mL, 0.234 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (122 mg, 0.234 mmol) was added, and the reaction was carried out at 70 °C for 16 hours.

After monitoring indicated completion of the reaction, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 25.9 mg of (3*S*,7*R*)-3-(4-(5-chloro-2-(4-(trifluoromethyl)-1*H-*1,2,3-triazol-1-yl)phenyl)-6-oxopyridazin-1(6*H*)-yl)-2⁴,2⁵-difluoro-7-methyl-1¹-(methyl-*d*₃)-1¹*H-*9-aza-1(5,4)-pyrazola-2(1,3)-benzenacyclononaphan-8-one as a white solid (yield: 16.7%). LC-MS: RT = 1.98 min, [M+H]⁺ = 662.07. ¹H NMR (400 MHz, DMSO-*d*₆) δ 9.22 (s, 1H), 9.19 (s, 1H), 7.94 (d, *J =* 1.4 Hz, 1H), 7.88 (d, *J =* 1.3 Hz, 2H), 7.81 (ddd, *J =* 11.2, 7.1, 2.0 Hz, 1H), 7.50 - 7.42 (m, 3H), 6.87 (d, *J =* 2.2 Hz, 1H), 6.12 (dd, *J =* 12.4, 3.3 Hz, 1H), 2.44-2.34 (m, 1H), 2.24-2.15 (m, 1H), 1.91-1.81 (m, 1H), 1.78 - 1.66 (m, 1H), 1.62-1.51(m, 1H), 1.43 - 1.32 (m, 1H), 1.02 (d, *J =* 6.7 Hz, 3H), 0.99-0.91 (m, 1H).

### Example 6

### Synthesis of (3S,7R)-3-(5-chloro-4-(3-chloro-2-fluoro-6-(4-(trifluoromethyl)-1H-1,2,3-triazol-1-yl)phenyl)-6-oxopyridazin-1(6H)-yl)-2⁴2⁵-difluoro-7-methyl-1¹-(methyl-d₃)-1¹H-9-aza-1(5,4)-pyrazola-2(1,3)-benzenacyclononaphan-8-one (6)

The specific synthetic route is as follows:

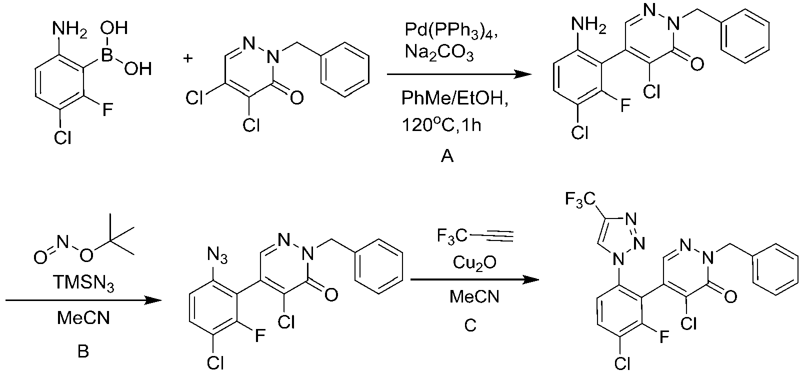

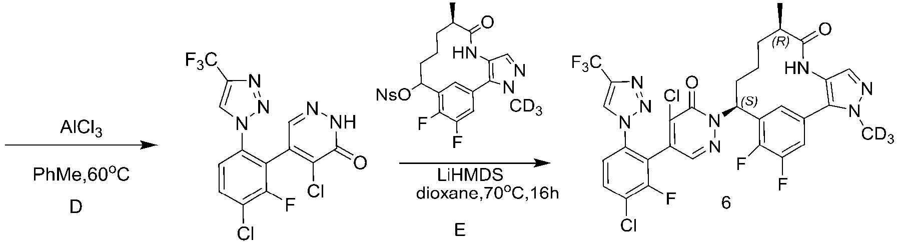

### Step A: Synthesis of 5-(6-amino-3-chloro-2-fluorophenyl)-2-benzyl-4-chloropyridazin-3(2H)-one

(6-Amino-3-chloro-2-fluorophenyl)boronic acid (1.80 g, 9.52 mmol, synthesis described in patent WO2017023992A1), 2-benzyl-4,5-dichloropyridazin-3(2*H*)-one (2.66 g, 10.5 mmol), and sodium carbonate (2.02 g, 19.1 mmol) were dissolved in toluene (16 mL) and ethanol (4 mL). Tetrakis(triphenylphosphine)palladium (549 mg, 0.476 mmol) was added, and the mixture was stirred at 120 °C for 1 hour under nitrogen protection.

After completion of the reaction, water and ethyl acetate were added for extraction. The organic phase was dried over anhydrous sodium sulfate and concentrated under reduced pressure. The residue was purified by silica gel column chromatography (eluent: ethyl acetate/n-hexane = 1/2) to afford 620 mg of 5-(6-amino-3-chloro-2-fluorophenyl)-2-benzyl-4-chloropyridazin-3(2*H*)-one as a pale yellow solid (yield: 17.9%). LCMS: RT = 2.29 min, [M+H]⁺ = 364.08.

### Step B: Synthesis of 5-(6-azido-3-chloro-2-fluorophenyl)-2-benzyl-4-chloropyridazin-3(2H)-one

5-(6-Amino-3-chloro-2-fluorophenyl)-2-benzyl-4-chloropyridazin-3(2*H*)-one (620 mg, 1.71 mmol) was dissolved in acetonitrile (10 mL). tert-Butyl nitrite (0.298 mL, 2.49 mmol) was added at 0 °C, followed by dropwise addition of trimethylsilyl azide (0.336 mL, 2.56 mmol). The reaction mixture was stirred at room temperature for 1 hour. After TLC monitoring indicated completion, the reaction mixture was poured into ice water and extracted with ethyl acetate. The organic phase was dried and concentrated to afford 500 mg of crude 5-(6-azido-3-chloro-2-fluorophenyl)-2-benzyl-4-chloropyridazin-3(2*H*)-one as a pale yellow foamy solid (yield: 75.2%), which was used directly in the next step. LCMS: RT = 2.17 min, [M+H]⁺ = 390.03.

### Step C: Synthesis of 2-benzyl-4-chloro-5-(3-chloro-2-fluoro-6-(4-(trifluoromethyl)-1H-1,2,3-triazol-1-yl)phenyl)pyridazin-3(2H)-one

Crude 5-(6-azido-3-chloro-2-fluorophenyl)-2-benzyl-4-chloropyridazin-3(2*H*)-one (500 mg, 1.28 mmol) was dissolved in acetonitrile (10 mL). Copper(I) oxide (56.1 mg, 0.384 mmol) was added. Trifluoropropyne gas was slowly bubbled into the mixture at room temperature until the reaction was complete.

Water and ethyl acetate were added for extraction. The organic phase was dried, concentrated, and the crude product was purified by silica gel column chromatography (eluent: ethyl acetate/n-hexane = 1/3) to afford 190 mg of 2-benzyl-4-chloro-5-(3-chloro-2-fluoro-6-(4-(trifluoromethyl)-1*H*-1,2,3-triazol-1-yl)phenyl)pyridazin-3(2*H*)-one as a pale yellow solid (yield: 30.7%). LCMS: RT = 2.10 min, [M+H]⁺ = 484.10.

### Step D: Synthesis of 4-chloro-5-(3-chloro-2-fluoro-6-(4-(trifluoromethyl)-1H-1,2,3-triazol-1-yl)phenyl)pyridazin-3(2H)-one

2-Benzyl-4-chloro-5-(3-chloro-2-fluoro-6-(4-(trifluoromethyl)-1*H*-1,2,3-triazol-1-yl)phenyl )pyridazin-3(2*H*)-one (190 mg, 0.496 mmol) was dissolved in toluene (8 mL). Aluminum trichloride (52.1 mg, 1.24 mmol) and p-toluenesulfonic acid monohydrate (1.67 g, 8.79 mmol) were added, and the reaction was carried out at 60 °C for 1 hour.

After TLC monitoring indicated completion, sodium bicarbonate solution and ethyl acetate were added for extraction. The organic phase was dried over anhydrous sodium sulfate and concentrated under reduced pressure. The residue was purified by column chromatography (eluent: ethyl acetate/n-hexane = 1/3) to afford 86.0 mg of 4-chloro-5-(3-chloro-2-fluoro-6-(4-(trifluoromethyl)-1*H*-1,2,3-triazol-1-yl)phenyl)pyridazin-3(2 H)-one as a pale yellow solid (yield: 44.1%). LCMS: RT = 1.82 min, [M+H]⁺ = 394.04. ¹H NMR (400 MHz, DMSO-*d*₆) δ 13.76 (s, 1H), 9.45 (d, *J =* 1.1 Hz, 1H), 8.23 - 8.14 (m, 1H), 7.92 (s, 1H), 7.89 (dd, *J =* 8.8, 1.6 Hz, 1H).

### Step E: Synthesis of (3S,7R)-3-(5-chloro-4-(3-chloro-2-fluoro-6-(4-(trifluoromethyl)-1H-1,2,3-triazol-1-yl)phenyl)-6-oxopyridazin-1(6H)-yl)-2⁴,2⁵-difluoro-7-methyl-1¹-(methyl-d₃)-1¹H -9-aza-1(5,4)-pyrazola-2(1,3)-benzenacyclononaphan-8-one

4-Chloro-5-(3-chloro-2-fluoro-6-(4-(trifluoromethyl)-1*H*-1,2,3-triazol-1-yl)phenyl)pyridazi n-3(2*H*)-one (100 mg, 0.254 mmol) was dissolved in dry 1,4-dioxane (8 mL). Lithium bis(trimethylsilyl)amide (1 M solution in tetrahydrofuran, 0.254 mL, 0.254 mmol) was added dropwise, and the resulting reaction mixture was stirred at room temperature for 30 minutes. Then, (3*R*,7*R*)-2⁴,2⁵-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (133 mg, 0.254 mmol) was added, and the reaction was carried out at 70 °C for 16 hours. After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 26.4 mg of (3*S*,7*R*)-3-(5-chloro-4-(3-chloro-2-fluoro-6-(4-(trifluoromethyl)-1*H*-1,2,3-triazol-1-yl)phenyl)-6-oxopyridazin-1 (6*H*)-yl)-2⁴,2⁵-difluoro-7-methyl-1¹-(methyl-*d*₃)-1¹*H*-9-aza-1(5,4)-pyrazola-2(1,3) -benzenacyclononaphan-8-one as a white solid (yield: 14.6%). LC-MS: RT = 2.05 min, [M+H]⁺ = 713.98. ¹H NMR (400 MHz, DMSO-*d*₆) δ 9.29 (d, *J =* 1.0 Hz, 1H), 9.25 (s, 1H), 8.22 - 8.14 (m, 1H), 7.92 - 7.80 (m, 3H), 7.48 (d, *J =* 5.9 Hz, 1H), 7.45 (s, 1H), 6.10 (dd, *J =* 12.6, 3.4 Hz, 1H), 2.45 - 2.38 (m, 1H), 2.28-2.18(m, 1H), 2.13 - 2.00 (m, 1H), 1.78-1.68 (m, 1H), 1.63 - 1.31 (m, 3H), 1.02(d, *J =* 6.4 Hz,3H).

### Example 7

### Synthesis of (3S,7R)-3-(4-(5-chloro-2-(4-(trifluoromethyl)-1H-1,2,3-triazol-1-yl)phenyl) -2-oxopyridin-1(2H)-yl)-2⁴,2⁵-difluoro-7-methyl-1¹-(methyl-d₃)-1¹H-9-aza-1(5,4)-pyrazola-2 (1,3)-benzenacyclononaphan-8-one (7)

The specific synthetic route is as follows:

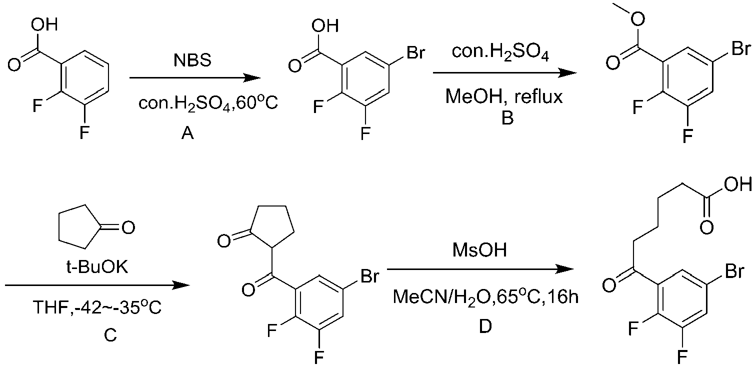

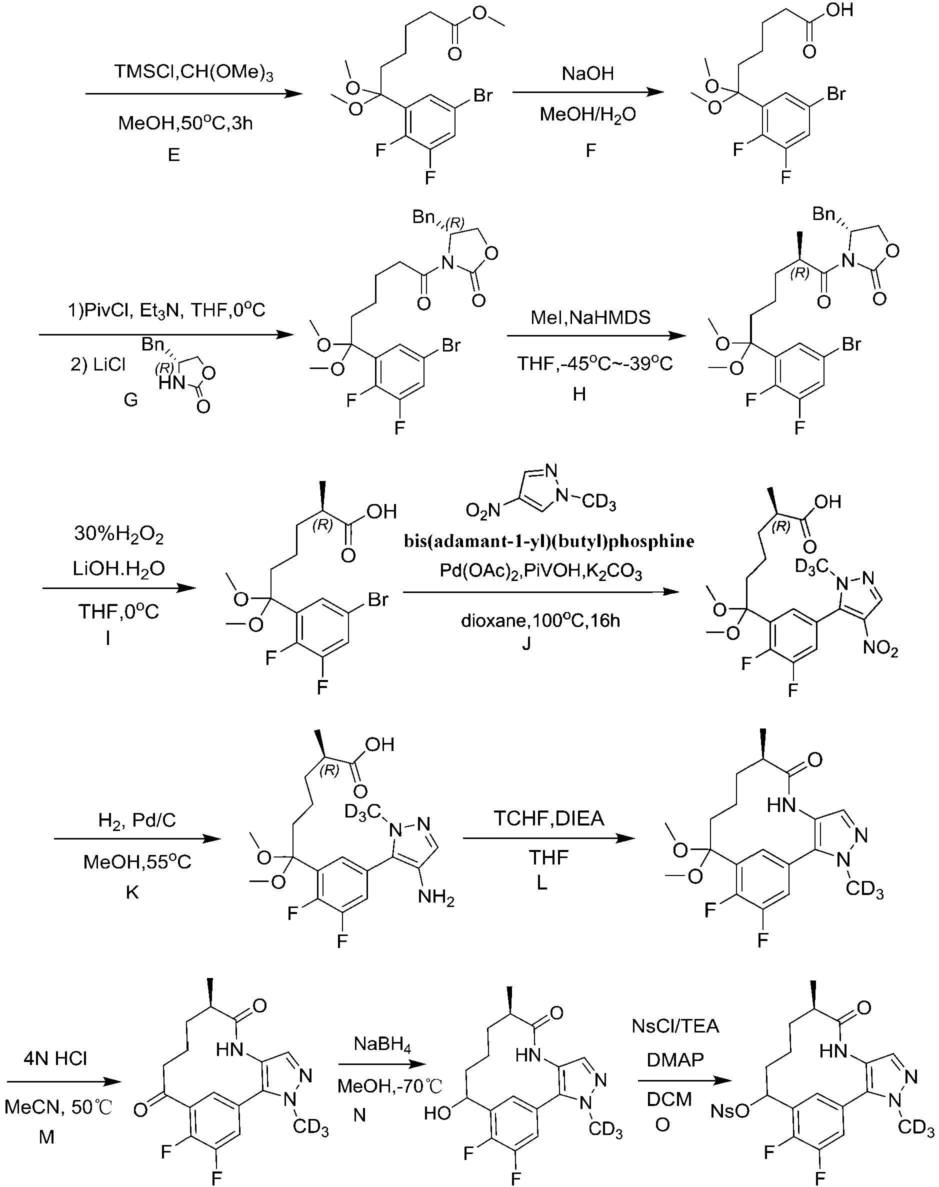

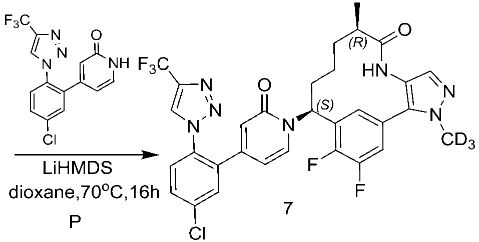

### Step A: Synthesis of 5-bromo-2,3-difluorobenzoic acid

2,3-Difluorobenzoic acid (50.0 g, 0.316 mol) and N-bromosuccinimide (61.9 g, 0.348 mol) were dissolved in concentrated sulfuric acid (150 mL). The mixture was stirred at 60 °C for 4 hours. After completion, the reaction mixture was cooled in an ice bath and slowly poured into ice water (2 L). A white solid precipitated, which was filtered off. The filter cake was washed with water three times and dried under reduced pressure to afford 66.4 g of 5-bromo-2,3-difluorobenzoic acid as a white solid (yield: 89.4%).

### Step B: Synthesis of methyl 5-bromo-2,3-difluorobenzoate

5-Bromo-2,3-difluorobenzoic acid (22.0 g, 93.2 mmol) was dissolved in methanol (220 mL). Concentrated sulfuric acid (10.1 mL) was added, and the mixture was stirred at 70 °C for 5 hours. After completion, the reaction mixture was slowly poured into ice water. A white solid precipitated, which was filtered off. The filter cake was washed with water and dried under reduced pressure to afford 23 g of methyl 5-bromo-2,3-difluorobenzoate as a pale yellow solid (yield: 98.7%).

### Step C: Synthesis of 2-(5-bromo-2,3-difluorobenzoyl)cyclopentan-1-one

At room temperature, cyclopentanone (47.2 g, 0.562 mol) and methyl 5-bromo-2,3-difluorobenzoate (117 g, 0.468 mol) were dissolved in tetrahydrofuran (800 mL). A solution of potassium tert-butoxide in tetrahydrofuran (1 M, 618 mL) was added dropwise at -42 °C, controlling the internal temperature not to exceed -35 °C. The reaction was stirred under nitrogen protection at this temperature for 2 hours. TLC monitoring indicated completion. The reaction was quenched with 2 N hydrochloric acid solution (200 mL). Most of the tetrahydrofuran was removed under reduced pressure, and the mixture was extracted with ethyl acetate (500 mL). The organic phase was dried over anhydrous sodium sulfate and concentrated under reduced pressure. The residue was purified by silica gel column chromatography (eluent: ethyl acetate/n-hexane = 1/9) to afford 110 g of 2-(5-bromo-2,3-difluorobenzoyl)cyclopentan-1-one as a pale yellow oil (yield: 77.8%).

### Step D: Synthesis of 6-(5-bromo-2,3-difluorophenyl)-6-oxohexanoic acid

At room temperature, 2-(5-bromo-2,3-difluorobenzoyl)cyclopentan-1-one (62.0 g, 0.205 mol) was added to 900 mL of a mixture of acetonitrile and water (3:2). The reaction was stirred under nitrogen protection at 65 °C for 16 hours. After cooling to room temperature, ethyl acetate (500 mL) was added for extraction. The organic phase was washed with water three times, dried, and concentrated under reduced pressure. The residue was purified by silica gel column chromatography (eluent: ethyl acetate/n-hexane = 2/11) to afford 14.5 g of 6-(5-bromo-2,3-difluorophenyl)-6-oxohexanoic acid as a white solid (yield: 22.1%). LCMS: RT = 1.97 min, [M-H]⁻ = 318.95.

### Step E: Synthesis of methyl 6-(5-bromo-2,3-difluorophenyl)-6,6-dimethoxyhexanoate

At room temperature, 6-(5-bromo-2,3-difluorophenyl)-6-oxohexanoic acid (14.5 g, 45.3 mmol) was added to methanol (150 mL). Trimethyl orthoformate (21.6 g, 204 mmol) and chlorotrimethylsilane (9.45 g, 86.9 mmol) were added sequentially. The reaction was stirred under nitrogen protection at 50 °C for 3 hours.

After completion, the reaction mixture was cooled to room temperature and quenched with saturated aqueous sodium bicarbonate solution. The mixture was extracted with dichloromethane (3 × 70 mL). The combined organic phases were washed with saturated brine (2 × 70 mL), dried over anhydrous sodium sulfate, and concentrated under reduced pressure to afford 16.4 g of crude methyl 6-(5-bromo-2,3-difluorophenyl)-6,6-dimethoxyhexanoate as a white solid (yield: 95.3%). LCMS: RT = 2.23 min, [M+H]⁺-CH₃O⁻ = 348.95.

### Step F: Synthesis of 6-(5-bromo-2,3-difluorophenyl)-6,6-dimethoxyhexanoic acid

At 0 °C, a 4.5 M sodium hydroxide solution (30 mL) was slowly added to a solution of methyl 6-(5-bromo-2,3-difluorophenyl)-6,6-dimethoxyhexanoate (16.4 g, 43.1 mmol) in methanol (150 mL). The reaction was stirred at room temperature for 2 hours.

After completion, the reaction mixture was neutralized with saturated ammonium chloride solution. The mixture was extracted with ethyl acetate (3 × 70 mL). The combined organic phases were washed with saturated brine (2 × 70 mL), dried over anhydrous sodium sulfate, and concentrated under reduced pressure. The crude product was triturated (n-hexane:ethyl acetate = 5:1) to afford 13.0 g of 6-(5-bromo-2,3-difluorophenyl)-6,6-dimethoxyhexanoic acid as a white solid (yield: 82.8%). LCMS: RT = 2.07 min, [M+H]⁺-CH₃O⁻ = 334.94.

### Step G: Synthesis of (R)-4-benzyl-3-(6-(5-bromo-2,3-difluorophenyl)-6,6-dimethoxy hexanoyl)oxazolidin-2-one

6-(5-Bromo-2,3-difluorophenyl)-6,6-dimethoxyhexanoic acid (13.0 g, 35.6 mmol) and triethylamine (12.4 mL, 89.0 mmol) were dissolved in anhydrous tetrahydrofuran (250 mL). Pivaloyl chloride (5.19 mL, 42.7 mmol) was added at 0 °C, and the mixture was stirred for 0.5 hours. Lithium chloride (1.79 g, 42.7 mmol) was added, and stirring was continued for 15 minutes. Then, (*R*)-4-benzyloxazolidin-2-one (7.57 g, 42.7 mmol) was added in batches, and the reaction was stirred at room temperature for 4 hours. After monitoring indicated completion, water was added to quench the reaction. Most of the tetrahydrofuran was removed under reduced pressure, and the mixture was extracted with ethyl acetate (2 × 40 mL). The combined organic phases were washed with saturated brine (3 × 80 mL), dried over anhydrous sodium sulfate, and purified by silica gel column chromatography (eluent: n-hexane:ethyl acetate = 3:1) to afford 16.6 g of (*R*)-4-benzyl-3-(6-(5-bromo-2,3-difluorophenyl)-6,6-dimethoxyhexanoyl)oxazolidin-2-one as a pale yellow oil (yield: 88.8%). LCMS: RT = 2.29 min, [M+H]⁺-CH₃O⁻ = 493.97.

### Step H: Synthesis of (R)-4-benzyl-3-((R)-6-(5-bromo-2,3-difluorophenyl)-6,6-dimethoxy-2-methylhexanoyl)oxazolidin-2-one

At -45 °C, iodomethane (4.34 mL, 69.6 mmol) was added to a solution of (*R*)-4-benzyl-3-((*R*)-6-(5-bromo-2,3-difluorophenyl)-6,6-dimethoxy-2-methylhexanoyl)oxazolidi n-2-one (16.6 g, 31.6 mmol) in tetrahydrofuran (200 mL). After stirring for 10 minutes, a solution of sodium bis(trimethylsilyl)amide in tetrahydrofuran (2 M, 20.6 mL) was added slowly, keeping the internal temperature below -39 °C. The reaction was stirred under nitrogen protection at -45 °C for 2 hours.

After monitoring indicated completion, the reaction was quenched with saturated ammonium chloride solution (50 mL). Most of the tetrahydrofuran was removed under reduced pressure, and the mixture was extracted with ethyl acetate (3 × 40 mL). The combined organic phases were dried over anhydrous sodium sulfate and concentrated to afford 17.0 g of crude (*R*)-4-benzyl-3-((*R*)-6-(5-bromo-2,3-difluorophenyl)-6,6-dimethoxy-2-methylhexanoyl)oxazolidi n-2-one as a pale yellow oil, which was used directly in the next step. LCMS: RT = 2.30 min, [M+H]⁺-CH₃O⁻ = 508.17.

### Step I: Synthesis of (R)-6-(5-bromo-2,3-difluorophenyl)-6,6-dimethoxy-2-methylhexanoic acid

Crude (*R*)-4-benzyl-3-((*R*)-6-(5-bromo-2,3-difluorophenyl)-6,6-dimethoxy-2-methylhexanoyl)oxazolidin-2-one (17.0 g, 31.5 mmol) was dissolved in tetrahydrofuran (150 mL). Aqueous hydrogen peroxide solution (30 wt%, 6.4 mL, 63.1 mmol) was added at 0 °C, and the mixture was stirred for 30 minutes. Finally, lithium hydroxide (2.65 g, 63.1 mmol) was added, and the reaction was stirred at room temperature for 2.0 hours.

After monitoring indicated completion, the reaction was quenched at 0 °C with an aqueous sodium sulfite solution (50 mL, 126 mmol). Most of the tetrahydrofuran was removed under reduced pressure, and the mixture was neutralized to pH being about 5 with citric acid solution. The mixture was extracted with ethyl acetate (3 × 30 mL). The combined organic phases were dried over anhydrous sodium sulfate and purified by silica gel column chromatography (eluent: n-hexane:ethyl acetate = 3:1) to afford 5.70 g of (*R*)-6-(5-bromo-2,3-difluorophenyl)-6,6-dimethoxy-2-methylhexanoic acid as a pale yellow oil (over two steps yield: 47.5%). LCMS: RT = 2.11 min, [M+H]⁺-CH₃O⁻ = 348.96.

### Step J: Synthesis of (R)-6-(2,3-difluoro-5-(1-(methyl-d₃)-4-nitro-1H-pyrazol-5-yl) phenyl)-6,6-dimethoxy-2-methylhexanoic acid

(*R*)-6-(5-Bromo-2,3-difluorophenyl)-6,6-dimethoxy-2-methylhexanoic acid (5.70 g, 15.0 mmol), 1-(methyl-*d*₃)-4-nitro-1*H*-pyrazole (1.95 g, 15.0 mmol), potassium carbonate (5.17 g, 37.5 mmol), and pivalic acid (306 mg, 3.00 mmol) were dissolved in anhydrous 1,4-dioxane (60 mL). Then, di(1-adamantyl)-n-butylphosphine (537 mg, 1.50 mmol) and palladium acetate (336 mg, 1.50 mmol) were added. After nitrogen exchange, the reaction was stirred at 100 °C for 16 hours. After monitoring indicated completion, the reaction mixture was cooled, water was added, and the pH was adjusted to about 6 with dilute hydrochloric acid. The mixture was extracted with ethyl acetate (3 × 40 mL). The combined organic phases were washed with saturated brine (2 × 50 mL), dried over anhydrous sodium sulfate, and purified by silica gel column chromatography (eluent: n-hexane:ethyl acetate = 2:1) to afford 3.57 g of (*R*)-6-(2,3-difluoro-5-(1-(methyl-*d*₃)-4-nitro-1*H*-pyrazol-5-yl)phenyl)-6,6-dimethoxy-2-methylhe xanoic acid as a pale yellow foamy solid (yield: 55.3%). LCMS: RT = 1.99 min, [M-H]⁻ = 429.08.

### Step K: Synthesis of (R)-6-(2,3-difluoro-5-(1-(methyl-d₃)-4-amino-1H-pyrazol-5-yl) phenyl)-6,6-dimethoxy-2-methylhexanoic acid

At room temperature, (*R*)-6-(2,3-difluoro-5-(1-(methyl-*d*₃)-4-nitro-1*H*-pyrazol-5-yl) phenyl)-6,6-dimethoxy-2-methylhexanoic acid (3.57 g, 8.31 mmol) was dissolved in methanol (70 mL). Pd/C (10% Pd with 50% water content, 714 mg) was added. After hydrogen exchange, a hydrogen balloon was attached, and the reaction was stirred at 50 °C for 6 hours. TLC monitoring indicated completion. The reaction mixture was cooled to room temperature, filtered through Celite, and the filtrate was dried and concentrated to afford 3.20 g of (*R*)-6-(2,3-difluoro-5-(1-(methyl-*d*₃)-4-amino-1*H*-pyrazol-5-yl)phenyl)-6,6-dimethoxy-2-methyl hexanoic acid as a pale red foamy solid (yield: 96.3%). LCMS: RT = 1.72 min, [M+H]⁺ = 401.13.

### Step L: Synthesis of (R)-2⁴,2⁵-difluoro-3,3-dimethoxy-7-methyl-1¹-(methyl-d₃)-1¹H-9-aza-1(5,4)-pyrazola-2(1,3)-benzenacyclononaphan-8-one

At room temperature, a solution of (*R*)-6-(2,3-difluoro-5-(1-(methyl-*d*₃)-4-amino-1*H-*pyrazol-5-yl)phenyl)-6,6-dimethoxy-2-methylhexanoic acid (3.20 g, 8.00 mmol) in tetrahydrofuran (300 mL) was slowly added dropwise to a mixture of N,N,N',N'-tetramethylchloroformamidinium hexafluorophosphate (3.03 g, 10.8 mmol) and N,N-diisopropylethylamine (3.61 g, 28.0 mmol) in tetrahydrofuran (100 mL). After the addition was complete, the reaction was stirred at room temperature for 1 hour.

TLC monitoring indicated completion. The reaction mixture was diluted with saturated ammonium chloride solution. Most of the tetrahydrofuran was removed under reduced pressure, and the mixture was extracted with ethyl acetate (3 × 30 mL). The combined organic phases were dried over anhydrous sodium sulfate and concentrated under reduced pressure. The crude product was purified by column chromatography (eluent: n-hexane:ethyl acetate = 1:9) to afford 2.86 g of (*R*)-2⁴,2⁵-difluoro-3,3-dimethoxy-7-methyl-1¹-(methyl-*d*₃)-1¹*H*-9-aza-1(5,4)-pyrazola-2(1,3)-benzenacyclononaphan-8-one as a pale yellow foamy solid (yield: 93.4%). LCMS: RT = 1.84 min, [M+H]⁺ = 383.13.

### Step M: Synthesis of (R)-2⁴,2⁵-difluoro-7-methyl-1¹-(methyl-d₃)-1¹H-9-aza-1(5,4)-pyrazola-2(1,3)-benzenacyclononaphan-3,8-dione

(*R*)-2⁴,2⁵-difluoro-3,3-dimethoxy-7-methyl-1¹-(methyl-*d*₃)-1¹*H*-9-aza-1(5,4)-pyrazola-2(1,3 )-benzenacyclononaphan-8-one (2.86 g, 7.46 mmol) was dissolved in acetonitrile (40 mL). A dilute hydrochloric acid solution (4 M in water, 6 mL) was added, and the reaction was stirred at 50 °C for 1 hour.

After monitoring indicated completion, the mixture was neutralized to neutrality with saturated sodium bicarbonate solution and extracted with ethyl acetate (2 × 30 mL). The combined organic phases were dried over anhydrous sodium sulfate and concentrated to afford 2.20 g of (*R*)-2⁴,2⁵-difluoro-7-methyl-1¹-(methyl-*d*₃)-1¹*H*-9-aza-1(5,4)-pyrazola-2(1,3)-benzenacyclonona phan-3,8-dione as a white solid (yield: 87.6%). LCMS: RT = 1.75 min, [M+H]⁺ = 337.11.

### Step N: Synthesis of (7R)-2⁴,2⁵-difluoro-3-hydroxy-7-methyl-1¹-(methyl-d₃)-1¹H-9-aza-1(5,4)-pyrazola-2(1,3)-benzenacyclononaphan-8-one

(*R*)-2⁴ 2⁵-difluoro-7-methyl-1¹-(methyl-*d*₃)-1¹*H*-9-aza-1(5,4)-pyrazola-2(1,3)-benzenacyclo nonaphan-3,8-dione (2.20 g, 6.54 mmol) was dissolved in anhydrous methanol (25 mL). Sodium borohydride (373 mg, 9.82 mmol) was added in portions at -70 °C, and the reaction was stirred at this temperature for 1 hour. After monitoring indicated completion, the reaction was quenched with saturated ammonium chloride solution and extracted with ethyl acetate (5 × 30 mL). The combined organic phases were dried over anhydrous sodium sulfate and concentrated to afford 1.52 g of (7*R*)-2⁴,25-difluoro-3-hydroxy-7-methyl-1¹-(methyl-*d*₃)-1¹*H*-9-aza-1(5,4)-pyrazola-2(1,3)-benzenacyclononaphan-8-one as a white solid (yield: 68.7%). LCMS: RT = 1.62 min, [M+H]⁺ = 339.04.

### Step O: Synthesis of (7R)-2⁴,2⁵-difluoro-7-methyl-1¹-(methyl-d₃)-8-oxo-1¹H-9-aza-1(5,4)-pyrazola-2(1,3)-benzenacyclononaphane-3-yl 4-nitrobenzenesulfonate

(7*R*)-2⁴,2⁵-difluoro-3-hydroxy-7-methyl-1'-(methyl-*d*₃)-1¹*H*-9-aza-1(5,4)-pyrazola-2(1,3)-b enzenacyclononaphan-8-one (1.52 g, 4.49 mmol) was dissolved in anhydrous dichloromethane (30 mL). Triethylamine (907 mg, 8.98 mmol) and 4-dimethylaminopyridine (109 mg, 0.898 mmol) were added sequentially, and the mixture was stirred for 10 minutes. Then, 4-nitrobenzenesulfonyl chloride (1.19 g, 5.39 mmol) was added in portions, and the reaction was stirred at room temperature for 6 hours.

After monitoring indicated completion, the mixture was diluted with sodium bicarbonate solution (30 mL) and extracted with dichloromethane (2 ×20 mL). The combined organic phases were dried over anhydrous sodium sulfate and concentrated under reduced pressure. The residue was purified by column chromatography (eluent: n-hexane:ethyl acetate = 1:15) to afford 1.34 g of (7*R*)-2⁴,2⁵-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate as a pale yellow solid (yield: 57.1%). LCMS: RT = 1.93 min, [M+H]⁺ = 524.02.

### Step P: Synthesis of (3S,7R)-3-(4-(5-chloro-2-(4-(trifluoromethyl)-1H-1,2,3-triazol-1-yl) phenyl)-2-oxopyridin-1(2H)-yl)-2⁴,2⁵-difluoro-7-methyl-1¹-(methyl-d₃)-1¹H-9-aza-1(5,4)-pyr azola-2(1,3)-benzenacyclononaphan-8-one

4-(5-Chloro-2-(4-(trifluoromethyl)-1*H*-1,2,3-triazol-1-yl)phenyl)pyridin-2(1*H*)-one (130 mg, 0.382 mmol) was dissolved in dry 1,4-dioxane (10 mL). Lithium bis(trimethylsilyl)amide (1 M solution in tetrahydrofuran, 0.382 mL, 0.382 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (200 mg, 0.382 mmol) was added, and the reaction was carried out at 70 °C for 16 hours.

After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 82.6 mg of (3*S*,7*R*)-3-(4-(5-chloro-2-(4-(trifluoromethyl)-1*H*-1,2,3-triazol-1-yl)phenyl)-2-oxopyridin-1(2*H*)-yl)-2⁴,2⁵-difluoro-7-methyl-1¹-(methyl-*d*₃)-1¹*H*-9-aza-1(5,4)-pyrazola-2(1,3)-b enzenacyclononaphan-8-one as a white solid (yield: 32.7%). LC-MS: RT = 1.98 min, [M+H]⁺ =661.08. ¹H NMR (400 MHz, DMSO-*d*₆) δ 9.26 (s, 1H), 9.08 (s, 1H), 7.90 - 7.78 (m, 4H), 7.52 (d, *J* = 6.0 Hz, 1H), 7.45 (s, 1H), 7.31 (d, *J =* 7.2 Hz, 1H), 6.30 (d, *J =* 2.0 Hz, 1H), 5.89 (d, *J =* 12.4 Hz, 1H), 5.67 (dd, *J =* 7.2, 2.1 Hz, 1H), 2.44-2.34 (m, 1H), 2.16 - 2.03 (m, 1H), 1.90-1.80 (m, 1H), 1.77 - 1.67 (m, 1H), 1.62-1.48 (m, 1H), 1.43-1.33 (m, 1H), 1.02 (d, *J =* 6.7 Hz, 3H) , 0.97-0.93 (m, 1H).

### Example 8

### Synthesis of (3S,7R)-3-(4-(5-chloro-2-(4-(trifluoromethyl)-1H-1,2,3-triazol-1-yl)phenyl) -3-chloro-2-oxopyridin-1(2H)-yl)-2⁴,2⁵-difluoro-7-methyl-1¹-(methyl-d₃)-1¹H-9-aza-1(5,4)-p yrazola-2(1,3)-benzenacyclononaphan-8-one (8)

The specific synthetic route is as follows:

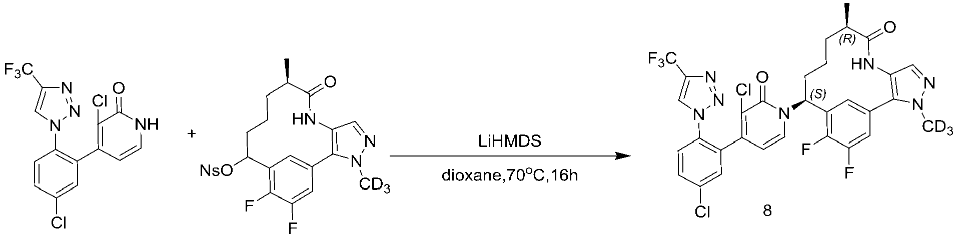

4-(5-Chloro-2-(4-(trifluoromethyl)-1*H*-1,2,3-triazol-1-yl)phenyl)-3-chloropyridin-2(1*H*)-on e (129 mg, 0.344 mmol) was dissolved in dry 1,4-dioxane (10 mL). Lithium bis(trimethylsilyl)amide (1 M solution in tetrahydrofuran, 0.181 mL, 0.181 mmol) was added dropwise, and the resulting reaction mixture was stirred at room temperature for 30 minutes. Then, (7*R*)-2⁴,2 ⁵-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (180 mg, 0.344 mmol) was added, and the reaction was carried out at 70 °C for 16 hours.

After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 50.6 mg of (3*S*,7*R*)-3-(4-(5-chloro-2-(4-(trifluoromethyl)-1*H*-1,2,3-triazol-1-yl)phenyl)-3-chloro-2-oxopyridin-1(2*H*)-yl)-2⁴,2⁵-difluoro-7-methyl-1¹-(methyl-*d*₃)-1¹*H*-9-aza-1(5,4)-pyrazol a-2(1,3)-benzenacyclononaphan-8-one as a white solid (yield: 21.2%). LC-MS: RT = 2.03 min, [M+H]⁺ =694.97.

¹H NMR (400 MHz, DMSO-*d*₆) δ 9.29 (s, 1H), 9.04 (s, 1H), 7.95 - 7.83 (m, 3H), 7.82 (t, *J* = 2.7 Hz,1H), 7.57-7.53 (m, 1H), 7.46 (s, 1H), 7.45-7.42 (m, 1H), 6.12 (dd, *J* = 7.2, 2.0 Hz, 1H), 5.93 (d, *J =* 12.0 Hz,1H), 2.46 - 2.36 (m, 1H), 2.25 - 2.09 (m, 1H), 1.98 - 1.82 (m, 1H), 1.79 - 1.67 (m, 1H), 1.65 - 1.50 (m, 1H), 1.45 - 1.33 (m,1H), 1.08 - 1.01 (m, 3H), 1.00 - 0.91 (m, 1H).

### Example 9

### Synthesis of (3S,7R)-3-(4-(5-chloro-2-(4-(trifluoromethyl)-1H-1,2,3-triazol-1-yl) phenyl)-3-fluoro-2-oxopyridin-1(2H)-yl)-2⁴,2⁵-difluoro-7-methyl-1¹-(methyl-d₃)-1¹H-9-aza-1 (5,4)-pyrazola-2(1,3)-benzenacyclononaphan-8-one (9)

The specific synthetic route is as follows:

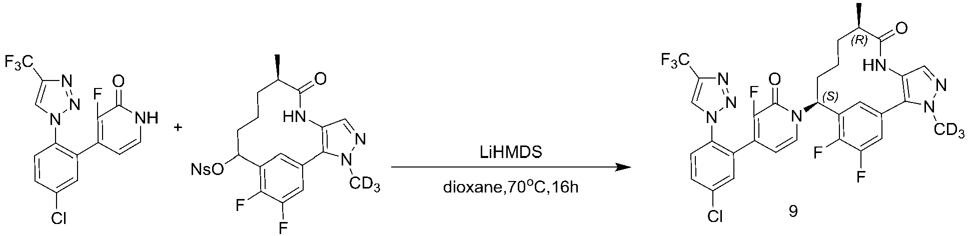

4-(5-Chloro-2-(4-(trifluoromethyl)-1*H*-1,2,3-triazol-1-yl)phenyl)-3-fluoropyridin-2(1*H*)-on e (65.0 mg, 0.181 mmol) was dissolved in dry 1,4-dioxane (8 mL). Lithium bis(trimethylsilyl)amide (1 M solution in tetrahydrofuran, 0.181 mL, 0.181 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (94.9 mg, 0.181 mmol) was added, and the reaction was carried out at 70 °C for 16 hours.

After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 24.9 mg of (3*S*,7*R*)-3-(4-(5-chloro-2-(4-(trifluoromethyl)-1*H*-1,2,3-triazol-1-yl)phenyl)-3-fluoro-2-oxopyridin-1(2*H*)-yl)-2⁴,2⁵-difluoro-7-methyl-1¹-(methyl-*d*₃)-1¹*H*-9-aza-1(5,4)-pyrazola -2(1,3)-benzenacyclononaphan-8-one as a white solid (yield: 20.3%). LC-MS: RT = 2.00 min, [M+H]⁺ =678.99.

¹H NMR (400 MHz, DMSO-*d*₆) δ 9.28 (s, 1H), 9.19 (s, 1H), 7.92-7.84 (m, 4H), 7.55 (d, *J =* 5.8 Hz, 1H), 7.46 (s, 1H), 7.27 (d, *J* = 7.3 Hz, 1H), 5.96-5.87(m, 2H), 2.44-2.35 (m, 1H), 2.20-2.08 (m, 1H), 1.95-1.85 (m, 1H), 1.78-1.68(m, 1H), 1.65-1.52 (m, 1H), 1.44-1.34 (m, 1H), 1.03 (d, *J =* 6.7 Hz, 3H) ,1.00-0.92 (m, 1H).

### Example 10

### Synthesis of (3S,7R)-3-(4-(5-chloro-2-(4-(difluoromethyl)-1H-1,2,3-triazol-1-yl)phenyl)-2-oxopyridin-1(2H)-yl)-2⁴,2⁵-difluoro-7-methyl-1¹-(methyl-d₃)-1¹H-9-aza-1(5,4)-pyrazola-2( 1,3)-benzenacyclononaphan-8-one (10)

The specific synthetic route is as follows:

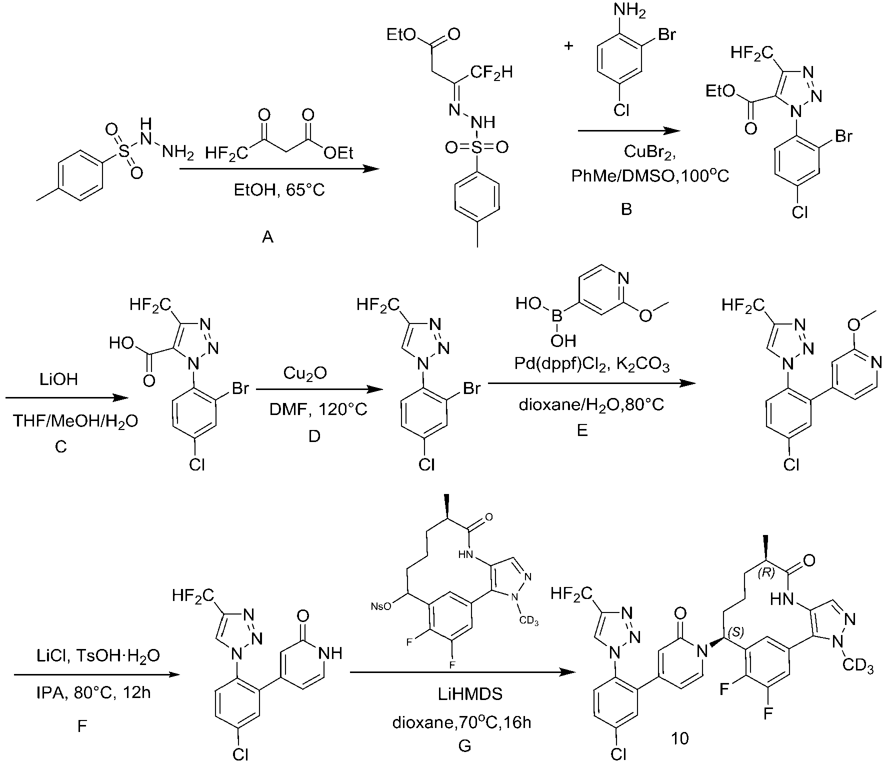

### Step A: Synthesis of ethyl (Z)-4,4-difluoro-3-((2-tosyl)hydrazono)butanoate

At room temperature, 4-methylbenzenesulfonohydrazide (10.0 g, 53.7 mmol) and ethyl 4,4-difluoro-3-oxobutanoate (9.80 g, 59.1 mmol) were dissolved in anhydrous ethanol (100 mL). The reaction was stirred under nitrogen protection at 65 °C for 5 hours.

TLC monitoring indicated completion. Most of the solvent was removed under reduced pressure. Water (200 mL) was added, and the mixture was extracted with ethyl acetate (2 × 60 mL). The combined organic phases were washed with saturated brine (3 × 100 mL), dried over anhydrous sodium sulfate, and concentrated to afford 16.2 g of ethyl (Z)-4,4-difluoro-3-((2-tosyl)hydrazono)butanoate as a white solid (yield: 90.5%). LCMS: RT = 1.73 min, [M+H]⁺ = 335.07.

### Step B: Synthesis of ethyl 1-(2-bromo-4-chlorophenyl)-4-(difluoromethyl)-1H-1,2,3-triazole-5-carboxylate

At room temperature, 2-bromo-4-chloroaniline (9.90 g, 48.5 mmol) and ethyl (Z)-4,4-difluoro-3-((2-tosyl)hydrazono)butanoate (16.2 g, 48.5 mmol) were added to toluene. Copper(II) bromide (2.10 g, 9.70 mmol) and dimethyl sulfoxide (48.2 mL, 679 mmol) were added. The reaction was stirred under nitrogen protection at 100 °C for 6 hours. TLC monitoring indicated completion. Most of the solvent was removed under reduced pressure. Water (200 mL) was added, and the mixture was extracted with ethyl acetate (2 × 60 mL). The combined organic phases were washed with saturated brine (3 × 100 mL), dried over anhydrous sodium sulfate, and purified by silica gel column chromatography (eluent: n-hexane:ethyl acetate = 3:1) to afford 13.1 g of ethyl 1-(2-bromo-4-chlorophenyl)-4-(difluoromethyl)-1*H*-1,2,3-triazole-5-carboxylate as a yellow solid (yield: 71.2%). LCMS: RT = 1.85 min, [M-H]- = 378.0.

### Step C: Synthesis of 1-(2-bromo-4-chlorophenyl)-4-(difluoromethyl)-1H-1,2,3-triazole-5-carboxylic acid

At 0 °C, ethyl 1-(2-bromo-4-chlorophenyl)-4-(difluoromethyl)-1*H*-1,2,3-triazole-5-carboxylate (13.1 g, 34.5 mmol) was added to a mixture of tetrahydrofuran/methanol/water (60 mL/20 mL/40 mL). Lithium hydroxide (1.70 g, 69.0 mmol) was added, and the reaction was stirred at room temperature for 16 hours.

LC-MS monitoring indicated complete consumption of the starting material. Most of the solvent was removed under reduced pressure. Dichloromethane (100 mL) was added, and the pH was adjusted to 4 with dilute hydrochloric acid. The mixture was extracted. The combined organic phases were washed with saturated brine (2 × 80 mL), dried over anhydrous sodium sulfate, and purified by silica gel column chromatography (eluent: dichloromethane: methanol = 15:1) to afford 9.10 g of 1-(2-bromo-4-chlorophenyl)-4-(difluoromethyl)-1*H*-1,2,3-triazole-5-carboxylic acid as a pale yellow solid (yield: 75.2%). LCMS: RT = 1.64 min, [M-H]⁻ = 350.11.

### Step D: Synthesis of 1-(2-bromo-4-chlorophenyl)-4-(difluoromethyl)-1H-1,2,3-triazole

At room temperature, 1-(2-bromo-4-chlorophenyl)-4-(difluoromethyl)-1*H*-1,2,3-triazole-5-carboxylic acid (9.10 g, 26.2 mmol) was added to N,N-dimethylformamide (50 mL). Copper(I) oxide (749 mg, 5.24 mmol) was added. The reaction was stirred under nitrogen protection at 120 °C for 1 hour.

LC-MS monitoring indicated completion. After cooling to room temperature, the reaction mixture was extracted with dichloromethane (3 × 80 mL). The combined organic phases were washed with saturated sodium chloride solution (4 × 100 mL), dried over anhydrous sodium sulfate, and purified by silica gel column chromatography (eluent: n-hexane:ethyl acetate = 4:1) to afford 5.00 g of 1-(2-bromo-4-chlorophenyl)-4-(difluoromethyl)-1*H*-1,2,3-triazole as a yellow solid (yield: 62.5%). LCMS: RT = 1.95 min, [M+H]+ = 307.97.

### Step E: Synthesis of 4-(5-chloro-2-(4-(difluoromethyl)-1H-1,2,3-triazol-1-yl)phenyl)-2-methoxypyridine

At room temperature, 1-(2-bromo-4-chlorophenyl)-4-(difluoromethyl)-1*H*-1,2,3-triazole (5.00 g, 16.2 mmol) and (2-methoxypyridin-4-yl)boronic acid (2.70 g, 17.8 mmol) were added to a mixture of dioxane/water (50 mL/10 mL). Potassium carbonate (5.60 g, 40.5 mmol) and [1,1'-bis(diphenylphosphino)ferrocene]palladium(II) dichloride (1.30 g, 1.80 mmol) were added. The reaction was stirred under nitrogen protection at 80 °C for 16 hours. LC-MS monitoring indicated completion. Most of the solvent was removed under reduced pressure. The mixture was extracted with ethyl acetate (3 × 30 mL). The combined organic phases were washed with saturated sodium chloride solution (4 × 100 mL), dried over anhydrous sodium sulfate, and purified by silica gel column chromatography (eluent: n-hexane:ethyl acetate = 3:1) to afford 2.70 g of 4-(5-chloro-2-(4-(difluoromethyl)-1*H*-1,2,3-triazol-1-yl)phenyl)-2-methoxypyridine as a yellow solid (yield: 50.2%). LCMS: RT = 2.04 min, [M+H]⁺ = 337.07.

### Step F: Synthesis of 4-(5-chloro-2-(4-(difluoromethyl)-1H-1,2,3-triazol-1-yl)phenyl) pyridin-2(1H)-one

4-(5-Chloro-2-(4-(difluoromethyl)-1*H*-1,2,3-triazol-1-yl)phenyl)-2-methoxypyridine (2.70 g, 8.00 mmol) was dissolved in isopropanol (40 mL). Lithium chloride (2.20 g, 51.6 mmol) and p-toluenesulfonic acid monohydrate (4.90 g, 25.8 mmol) were added. The reaction was stirred at 80 °C overnight. After TLC showed no starting material remained, triethylamine (5 mL) was added to neutralize the reaction. The mixture was concentrated under reduced pressure. The residue was dissolved in ethyl acetate (100 mL) and washed with saturated brine (3 × 50 mL). The organic phase was dried over anhydrous sodium sulfate and concentrated under reduced pressure. The residue was purified by silica gel column chromatography (eluent: ethyl acetate/n-hexane = 3/1) to afford 1.50 g of 4-(5-chloro-2-(4-(difluoromethyl)-1*H*-1,2,3-triazol-1-yl)phenyl)pyridin-2(1*H*)-one as a white solid (yield: 58.0%). LCMS: RT = 1.73 min, [M+H]⁺ = 323.08.

### Step G: Synthesis of (3S,7R)-3-(4-(5-chloro-2-(4-(difluoromethyl)-1H-1,2,3-triazol-1-yl) phenyl)-2-oxopyridin-1(2H)-yl)-2⁴,2⁵-difluoro-7-methyl-1¹-(methyl-d₃)-1¹H-9-aza-1(5,4)-pyr azola-2(1,3)-benzenacyclononaphan-8-one

4-(5-Chloro-2-(4-(difluoromethyl)-1*H*-1,2,3-triazol-1-yl)phenyl)pyridin-2(1*H*)-one (100 mg, 0.310 mmol) was dissolved in dry 1,4-dioxane (5 mL). Lithium bis(trimethylsilyl)amide (1 M solution in tetrahydrofuran, 0.310 mL, 0.310 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (162 mg, 0.310 mmol) was added. The reaction temperature was raised to 70 °C and stirred at this temperature for 16 hours.

TLC monitoring indicated completion. The mixture was cooled to room temperature. Saturated aqueous ammonium chloride solution (4 mL) and ethyl acetate (20 mL) were added for extraction. The organic phase was washed with saturated brine (3 × 10 mL), dried over anhydrous sodium sulfate, and concentrated under reduced pressure. The residue was purified by silica gel column chromatography (eluent: ethyl acetate/petroleum ether = 4/1), followed by purification using preparative high-performance liquid chromatography to afford 33.0 mg of (3*S*,7*R*)-3-(4-(5-chloro-2-(4-(difluoromethyl)-1*H*-1,2,3-triazol-1-yl)phenyl)-2-oxopyridin-1(2*H*)-yl)-2⁴,2⁵-difluoro-7-methyl-1¹-(methyl-*d*₃)-1¹*H*-9-aza-1(5,4)-pyrazola-2(1,3)-benzenacyclononap han-8-one as a white solid (yield: 15.0%). LCMS: RT = 1.93 min, [M+H] ⁺ = 643.13. ¹H NMR (400 MHz, DMSO-*d*₆) δ 9.25 (s, 1H), 8.73 (s, 1H), 7.90 - 7.70 (m, 4H), 7.52 (d, *J =* 5.9 Hz, 1H), 7.45 (s, 1H), 7.39 - 7.06 (m, 2H), 6.29 (d, *J =* 2.0 Hz, 1H), 5.90 (d, *J =* 12.2 Hz, 1H), 5.69 (dd, *J* = 7.2, 2.1 Hz, 1H), 2.46 - 2.33 (m, 1H), 2.17 - 2.01 (m, 1H), 1.95 - 1.80 (m, 1H), 1.80 - 1.64 (m, 1H), 1.63 - 1.46 (m, 1H), 1.45 - 1.31 (m, 1H), 1.02 (d, *J* = 6.7 Hz, 3H), 1.01 - 0.85 (m, 1H).

### Example 11

### Synthesis of (3S,7R)-3-(4-(3-chloro-2-fluoro-6-(4-(trifluoromethyl)-1H-1,2,3-triazol-1-yl)phenyl)-6-oxopyrimidin-1(6H)-yl)-2⁴,2⁵-difluoro-7-methyl-1¹-difluoromethyl-1¹H-9-aza-1 (5,4)-pyrazola-2(1,3)-benzenacyclononaphan-8-one (11)

The specific synthetic route is as follows:

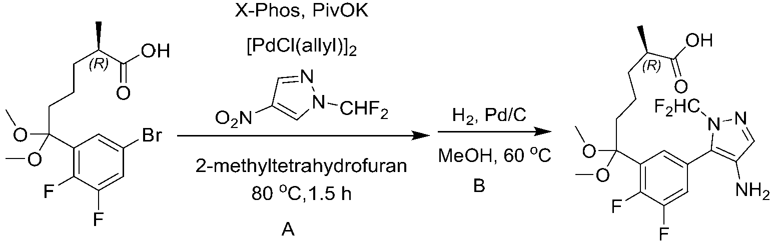

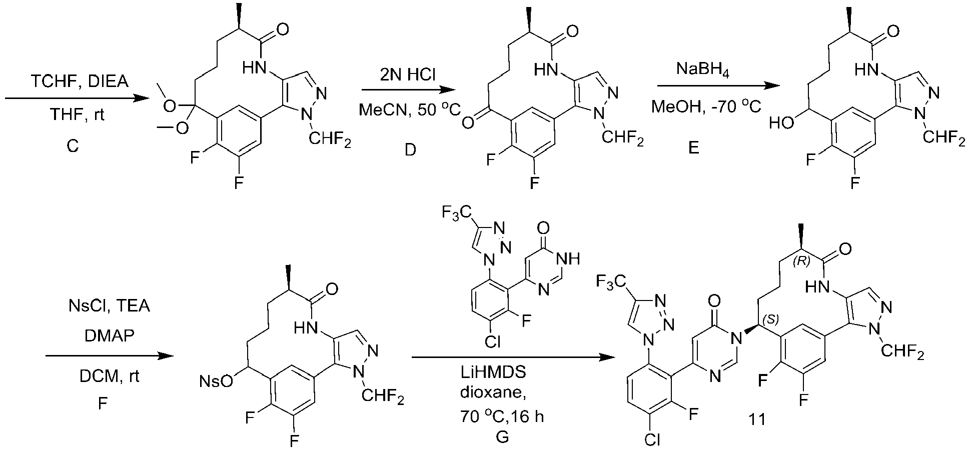

### Step A: Synthesis of (R)-6-(2,3-difluoro-5-(1-difluoromethyl-4-nitro-1H-pyrazol-5-yl) phenyl)-6,6-dimethoxy-2-methylhexanoic acid

[Pd(allyl)Cl]₂ (318 mg, 0.87 mmol) and XPhos (619 mg, 1.3 mmol) were dissolved in anhydrous 2-methyltetrahydrofuran (50 mL). After stirring at room temperature for 0.5 hours, (*R*)-6-(5-bromo-2,3-difluorophenyl)-6,6-dimethoxy-2-methylhexanoic acid (3 g, 8.67 mmol), 1-difluoromethyl-4-nitro-1*H*-pyrazole (1.6 g, 9.54 mmol), and potassium pivalate (1.8 g, 13 mmol) were added. After nitrogen exchange, the reaction was stirred at 80 °C for 1.5 hours. After monitoring indicated completion, the reaction mixture was cooled, water was added, and the pH was adjusted to about 6 with dilute hydrochloric acid. The mixture was extracted with ethyl acetate (3 × 40 mL). The combined organic phases were washed with saturated sodium chloride solution (2 × 50 mL), dried over anhydrous sodium sulfate, and purified by silica gel column chromatography (eluent: n-hexane/ethyl acetate = 2/1) to afford 3.2 g of (*R*)-6-(2,3-difluoro-5-(1-difluoromethyl-4-nitro-1*H*-pyrazol-5-yl)phenyl)-6,6-dimethoxy-2-meth ylhexanoic acid as a pale yellow foamy solid (yield: 80%). LCMS: RT = 2.04 min, [M-H]⁻ = 462.11.

### Step B: Synthesis of (R)-6-(2,3-difluoro-5-(1-difluoromethyl-4-amino-1H-pyrazol-5-yl) phenyl)-6,6-dimethoxy-2-methylhexanoic acid

At room temperature, (*R*)-6-(2,3-difluoro-5-(1-difluoromethyl-4-nitro-1*H*-pyrazol-5-yl) phenyl)-6,6-dimethoxy-2-methylhexanoic acid (3.2 g, 6.89 mmol) was dissolved in methanol (100 mL). Pd/C (10% Pd with 50% water content, 1.28 g) was added. After hydrogen exchange, a hydrogen balloon was attached, and the reaction was stirred at 60 °C for 2 hours. TLC monitoring indicated completion. The reaction mixture was cooled to room temperature, filtered through Celite, and the filtrate was dried and concentrated to afford 3 g of (*R*)-6-(2,3-difluoro-5-(1-difluoromethyl-4-amino-1*H*-pyrazol-5-yl)phenyl)-6,6-dimethoxy-2-met hylhexanoic acid as a pale yellow foamy solid. LCMS: RT = 1.81 min, [M+H]⁺ = 434.13.

### Step C: Synthesis of (R)-2⁴,2⁵-difluoro-3,3-dimethoxy-7-methyl-1¹-difluoromethyl-1¹H-9-aza-1(5,4)-pyrazola-2(1,3)-benzenacyclononaphan-8-one

At room temperature, a solution of (*R*)-6-(2,3-difluoro-5-(1-difluoromethyl-4-amino-1*H-*pyrazol-5-yl)phenyl)-6,6-dimethoxy-2-methylhexanoic acid (3 g, 6.91 mmol) in tetrahydrofuran (600 mL) was slowly added dropwise to a mixture of N,N,N',N'-tetramethylchloroformamidinium hexafluorophosphate (3.87 g, 13.82 mmol) and N,N-diisopropylethylamine (1.78 g, 13.84 mmol) in tetrahydrofuran (500 mL). After the addition was complete, the reaction was stirred at room temperature for 1 hour.

TLC monitoring indicated completion. The reaction mixture was diluted with saturated ammonium chloride solution. Most of the tetrahydrofuran was removed under reduced pressure, and the mixture was extracted with ethyl acetate (3 × 30 mL). The combined organic phases were dried over anhydrous sodium sulfate and concentrated under reduced pressure. The crude product was purified by column chromatography (eluent: n-hexane:ethyl acetate = 1:10) to afford 2.8 g of (*R*)-2⁴2⁵-difluoro-3,3-dimethoxy-7-methyl-1¹-difluoromethyl-1¹*H*-9-aza-1(5,4)-pyrazola-2(1,3)-benzenacyclononaphan-8-one as a pale yellow foamy solid. LCMS: RT = 1.88 min, [M+H]⁺ = 416.13.

### Step D: Synthesis of (R)-2⁴,2⁵-difluoro-7-methyl-1¹-difluoromethyl-1¹H-9-aza-1(5,4)-pyrazola-2(1,3)-benzenacyclononaphan-3,8-dione

(*R*)-2⁴,2⁵-difluoro-3,3-dimethoxy-7-methyl-1¹-difluoromethyl-1¹*H*-9-aza-1(5,4)-pyrazola-2( 1,3)-benzenacyclononaphan-8-one (2.8 g, 6.75 mmol) was dissolved in acetonitrile (40 mL). A dilute hydrochloric acid solution (2 M in water, 10 mL) was added, and the reaction was stirred at 50 °C for 3 hours.

After monitoring indicated completion, the mixture was neutralized to pH 7 with saturated sodium bicarbonate solution and extracted with ethyl acetate (2 ×30 mL). The combined organic phases were dried over anhydrous sodium sulfate and concentrated to afford 1.92 g of (*R*)-2⁴,2⁵-difluoro-7-methyl-1¹-difluoromethyl-1¹*H*-9-aza-1(5,4)-pyrazola-2(1,3)-benzenacyclono naphan-3,8-dione as a white solid (yield: 78.3%). LCMS: RT = 1.75 min, [M+H]⁺ = 370.14.

### Step E: Synthesis of (7R)-2⁴,2⁵-difluoro-3-hydroxy-7-methyl-1¹-difluoromethyl-1¹H-9-aza-1(5,4)-pyrazola-2(1,3)-benzenacyclononaphan-8-one

(*R*)-2^{4,}2⁵-difluoro-7-methyl-1¹-difluoromethyl-1¹*H*-9-aza-1(5,4)-pyrazola-2(1,3)-benzenacy clononaphan-3,8-dione (1.92 g, 5.19 mmol) was dissolved in anhydrous methanol (125 mL). Sodium borohydride (200 mg, 5.19 mmol) was added in portions at -70 °C, and the reaction was stirred at this temperature for 1 hour.

After monitoring indicated completion, the reaction was quenched with saturated ammonium chloride solution and extracted with ethyl acetate (5 × 30 mL). The combined organic phases were dried over anhydrous sodium sulfate and concentrated to afford 1.8 g of (7*R*)-2⁴,2⁵-difluoro-3-hydroxy-7-methyl-1¹-difluoromethyl-1¹*H*-9-aza-1(5,4)-pyrazola-2(1,3)-ben zenacyclononaphan-8-one as a white solid (yield: 93%). LCMS: RT = 1.65 min, [M+H]⁺ = 372.11.

### Step F: Synthesis of (7R)-2⁴,2⁵-difluoro-7-methyl-1¹-difluoromethyl-8-oxo-1¹H-9-aza-1(5,4)-pyrazola-2(1,3)-benzenacyclononaphane-3-yl 4-nitrobenzenesulfonate

(7*R*)-2⁴,2⁵-difluoro-3-hydroxy-7-methyl-1¹-difluoromethyl-1¹*H*-9-aza-1(5,4)-pyrazola-2(1,3 )-benzenacyclononaphan-8-one (400 mg, 1.08 mmol) was dissolved in anhydrous dichloromethane (30 mL). Triethylamine (218 mg, 2.15 mmol) and 4-dimethylaminopyridine (131 mg, 1.08 mmol) were added sequentially, and the mixture was stirred for 10 minutes. Then, 4-nitrobenzenesulfonyl chloride (357 mg, 1.61 mmol) was added in portions, and the reaction was stirred at room temperature for 10 minutes.

After monitoring indicated completion, the mixture was diluted with sodium bicarbonate solution (30 mL) and extracted with dichloromethane (2 ×20 mL). The combined organic phases were dried over anhydrous sodium sulfate and concentrated under reduced pressure. The residue was purified by column chromatography (eluent: n-hexane/ethyl acetate = 1/10) to afford 500 mg of (7*R*)-2⁴,2⁵-difluoro-7-methyl-1¹-difluoromethyl-8-oxo-1¹*H*-9-aza-1(5,4)-pyrazola-2(1,3)-benzenacyclononaphane-3-yl 4-nitrobenzenesulfonate as a pale yellow solid (yield: 83%). LCMS: RT = 1.98 min, [M+H]⁺ = 557.02.

### Step G: Synthesis of (3S,7R)-3-(4-(3-chloro-2-fluoro-6-(4-(trifluoromethyl)-1H-1,2,3-triazol-1-yl)phenyl)-6-oxopyrimidin-1(6H)-yl)-2⁴2⁵-difluoro-7-methyl-1¹-difluoromethyl-1¹ H-9-aza-1(5,4)-pyrazola-2(1,3)-benzenacyclononaphan-8-one

6-(3-Chloro-2-fluoro-6-(4-(trifluoromethyl)-1*H*-1,2,3-triazol-1-yl)phenyl)pyrimidin-4(3*H*)-one (130 mg, 0.36 mmol) was dissolved in dry 1,4-dioxane (15 mL). Lithium bis(trimethylsilyl)amide (1 M solution in tetrahydrofuran, 0.36 mL, 0.36 mmol) was added dropwise, and the resulting reaction mixture was stirred at room temperature for 30 minutes. Then, (7*R*)-2⁴,2⁵-difluoro-7-methyl-1¹-difluoromethyl-8-oxo-1¹*H*-9-aza-1(5,4)-pyrazola-2(1,3)-benzenacyclononaphane-3-yl 4-nitrobenzenesulfonate (200 mg, 0.36 mmol) was added, and the reaction was carried out at 70 °C for 16 hours.

After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 62 mg of (3*S*,7*R*)-3-(4-(3-chloro-2-fluoro-6-(4-(trifluoromethyl)-1*H*-1,2,3-triazol-1-yl) phenyl)-6-oxopyrimidin-1(6*H*)-yl)-2⁴2⁵-difluoro-7-methyl-1¹-difluoromethyl-1¹*H*-9-aza-1(5,4)-pyrazola-2(1,3)-benzenacyclononaphan-8-one as a white solid (yield: 24%). LC-MS: RT = 2.04 min, [M+H]⁺ = 712.94. ¹H NMR (400 MHz, DMSO-*d*₆) δ 9.42 (s, 1H), 9.15 (s, 1H), 8.33 (s, 1H), 8.14-7.86 (m, 3H), 7.78-7.74 (m, 1H), 7.60 (dt, *J =* 18.1, 7.0 Hz, 2H), 6.70 (s, 1H), 5.63 (d, *J =* 12.4 Hz, 1H), 2.48-2.37 (m, 2H), 2.04-1.90 (m, 1H), 1.83-1.72 (m, 1H), 1.52-1.35 (m, 2H), 1.11-0.95 (m, 4H).

### Example 12

### Synthesis of (3R,7R)-3-(4-(5-chloro-2-(4-(trifluoromethyl)-1H-1,2,3-triazol-1-yl)phenyl) -6-oxopyrimidin-1(6H)-yl)-24,2⁵-difluoro-7-methyl-1¹-difluoromethyl-1¹H-9-aza-1(5,4)-pyr azola-2(1,3)-benzenacyclononaphan-8-one (12)

The specific synthetic route is as follows:

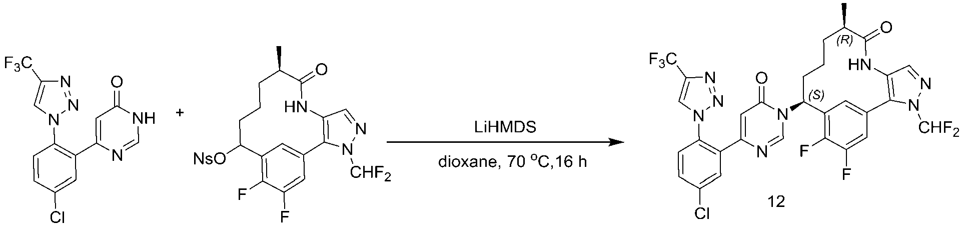

6-(5-Chloro-2-(4-(trifluoromethyl)-1*H*-1,2,3-triazol-1-yl)phenyl)pyrimidin-4(3*H*)-one (123 mg, 0.36 mmol) was dissolved in dry 1,4-dioxane (15 mL). Lithium bis(trimethylsilyl)amide (1 M solution in tetrahydrofuran, 0.36 mL, 0.36 mmol) was added dropwise, and the resulting reaction mixture was stirred at room temperature for 30 minutes. Then, (7*R*)-2⁴,2⁵-difluoro-7-methyl-1¹-difluoromethyl-8-oxo-1¹*H*-9-aza-1(5,4)-pyrazola-2(1,3)-benzena cyclononaphane-3-yl 4-nitrobenzenesulfonate (200 mg, 0.36 mmol) was added, and the reaction was carried out at 70 °C for 16 hours.

After monitoring indicated completion, the mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 50 mg of (3*R*,7*R*)-3-(4-(5-chloro-2-(4-(trifluoromethyl)-1*H*-1,2,3-triazol-1-yl)phenyl)-6-oxopyrimidin-1(6*H*)-yl)-2^{4,}2⁵-difluoro-7-methyl-1¹-difluoromethyl-1¹*H*-9-aza-1(5,4)-pyrazola-2( 1,3)-benzenacyclononaphan-8-one as a white solid (yield: 20%). LC-MS: RT = 1.98 min, [M+H]⁺ =678.96. ¹H NMR (400 MHz, DMSO-*d*₆) δ 9.43 (s, 1H), 8.67 (s, 1H), 8.39 (s, 1H), 8.08-7.77 (m, 3H), 7.68 (dd, *J* = 8.8, 1.4 Hz, 1H), 7.64-7.55 (m, 2H), 6.65 (s, 1H), 5.64 (d, *J =* 11.8 Hz, 1H), 2.48-2.38 (m, 2H), 2.04-1.92 (m, 1H), 1.84-1.73 (m, 1H), 1.51-1.37 (m, 2H), 1.12-0.99 (m, 4H).

### Example 13

### Synthesis of (3S,7R)-3-(4-(3-chloro-6-(4-chloro-1H-1,2,3-triazol-1-yl)-2-fluorophenyl)-6-oxopyrimidin-1(6H)-yl)-2⁴,2⁵-difluoro-7-methyl-1¹-difluoromethyl-1¹H-9-aza-1(5,4)-pyraz ola-2(1,3)-benzenacyclononaphan-8-one (13)

The specific synthetic route is as follows:

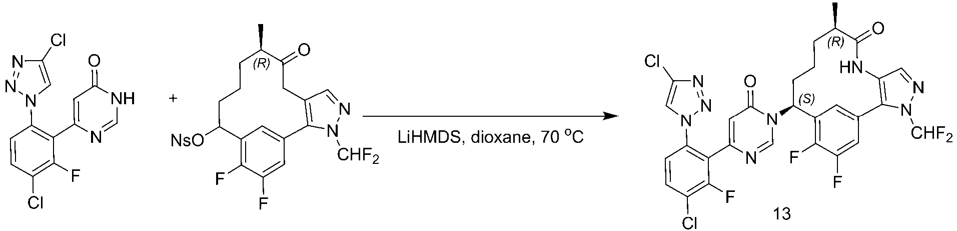

4-(3-Chloro-6-(4-chloro-1*H*-1,2,3-triazol-1-yl)-2-fluorophenyl)pyrimidin-2(1*H*)-one (118 mg, 0.36 mmol) was dissolved in dry 1,4-dioxane (15 mL). Lithium bis(trimethylsilyl)amide (1 M solution in tetrahydrofuran, 0.36 mL, 0.36 mmol) was added dropwise, and the resulting reaction mixture was stirred at room temperature for 30 minutes. Then, (7*R*)-2⁴,2⁵-difluoro-7-methyl-1¹-difluoromethyl-8-oxo-1¹*H*-9-aza-1(5,4)-pyrazola-2(1,3)-benzena cyclononaphan-3-yl 4-nitrobenzenesulfonate (200 mg, 0.36 mmol) was added, and the reaction mixture was stirred at 70 °C for 24 hours.

After TLC monitoring indicated completion, the mixture was cooled to room temperature. Saturated aqueous ammonium chloride solution (25 mL) and ethyl acetate (30 mL) were added. The mixture was separated, and the organic phase was washed with saturated brine, dried over anhydrous sodium sulfate, and concentrated under reduced pressure. The residue was purified by silica gel column chromatography (eluent: ethyl acetate/petroleum ether = 10/1), followed by purification using preparative high-performance liquid chromatography to afford 45 mg of (3*S*,7*R*)-3-(4-(3-chloro-6-(4-chloro-1*H*-1,2,3-triazol-1-yl)-2-fluorophenyl)-6-oxopyrimidin-1(6*H* )-yl)-2⁴2⁵-difluoro-7-methyl-1¹-difluoromethyl-1¹*H*-9-aza-1(5,4)-pyrazola-2(1,3)-benzenacyclon onaphan-8-one as a white solid (yield: 18%). LC-MS: RT = 1.98 min, [M+H]⁺ =678.96. ¹H NMR (400 MHz, DMSO-*d*₆) δ 9.43 (s, 1H), 8.67 (s, 1H), 8.39 (s, 1H), 8.08-7.77 (m, 3H), 7.68 (dd, *J* = 8.8, 1.4 Hz, 1H), 7.64-7.55 (m, 2H), 6.65 (s, 1H), 5.64 (d, *J* = 11.8 Hz, 1H), 2.48-2.38 (m, 2H), 2.04-1.92 (m, 1H), 1.84-1.73 (m, 1H), 1.51-1.37 (m, 2H), 1.12-0.99 (m, 4H).

### Example 14

### Synthesis of (3S,7R)-3-(4-(5-chloro-2-(4-chloro-1H-1,2,3-triazol-1-yl)phenyl)-6-oxopyrimidin-1(6H)-yl)-2⁴,2⁵-difluoro-7-methyl-1¹-difluoromethyl-1¹H-9-aza-1(5,4)-pyrazol a-2(1,3)-benzenacyclononaphan-8-one (14)

The specific synthetic route is as follows:

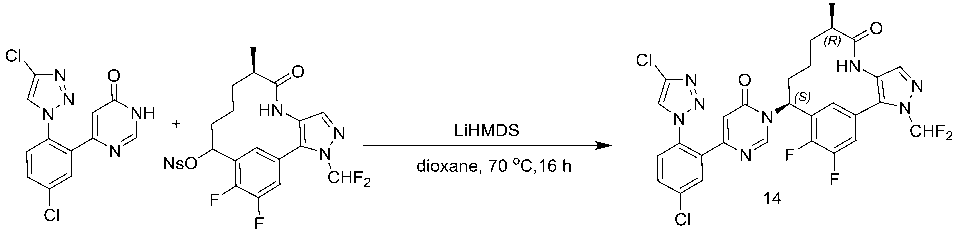

6-(5-Chloro-2-(4-chloro-1*H*-1,2,3-triazol-1-yl)phenyl)pyrimidin-4(3*H*)-one (110 mg, 0.36 mmol) was dissolved in dry 1,4-dioxane (15 mL). Lithium bis(trimethylsilyl)amide (1 M solution in tetrahydrofuran, 0.36 mL, 0.36 mmol) was added dropwise, and the resulting reaction mixture was stirred at room temperature for 30 minutes. Then, (7*R*)-2⁴,2⁵-difluoro-7-methyl-1¹-difluoromethyl-8-oxo-1¹*H*-9-aza-1(5,4)-pyrazola-2(1,3)-benzena cyclononaphane-3-yl 4-nitrobenzenesulfonate (200 mg, 0.36 mmol) was added, and the reaction was carried out at 70 °C for 16 hours.

After monitoring indicated completion, the mixture was cooled to room temperature. Saturated aqueous ammonium chloride solution (25 mL) and ethyl acetate (30 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 42 mg of (3*S*,7*R*)-3-(4-(5-chloro-2-(4-chloro-1*H*-1,2,3-triazol-1-yl)phenyl)-6-oxopyrimidin-1(6*H*)-yl)-2⁴2⁵-difluoro-7-methyl-1¹-difluoromethyl-1¹*H*-9-aza-1(5,4)-pyrazola-2( 1,3)-benzenacyclononaphan-8-one as a white solid (yield: 18%). LC-MS: RT = 2.00 min, [M+H]⁺ = 660.95. ¹H NMR (400 MHz, DMSO-*d*₆) δ 9.42 (s, 1H), 8.71 (s, 1H), 8.34 (s, 1H), 8.08-7.87 (m, 3H), 7.82 (dd, *J =* 8.5, 2.4 Hz, 1H), 7.75 (t, *J =* 7.8 Hz, 1H), 7.60 (dd, *J =* 11.7, 6.5 Hz, 2H), 6.45 (s, 1H), 5.70-5.54 (m, 1H), 2.49-2.37 (m, 2H), 2.01-1.89 (m, 1H), 1.1.82-1.71 (m, 1H), 1.55-1.37 (m, 2H), 1.10-0.97 (m, 4H).

### Example 15

### Synthesis of (3S,7R)-3-(4-chloro-2-fluoro-6-(4-(trifluoromethyl)-1H-1,2,3-triazol-1-yl) phenyl)-2-oxopyridin-1(2H)-yl)-2⁴,2⁵-difluoro-7-methyl-1¹-(methyl-d₃)-1¹H-9-aza-1(5,4)-pyr azola-2(1,3)-benzenacyclononaphan-8-one (15)

The specific synthetic route is as follows:

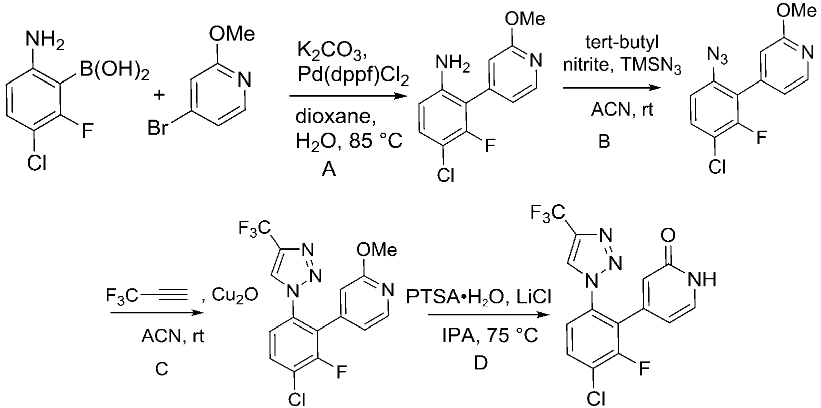

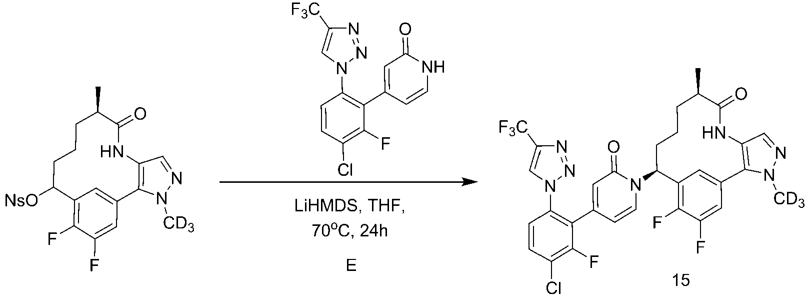

### Step A: Synthesis of 4-chloro-3-fluoro-2-(2-methoxypyridin-4-yl)aniline

At room temperature, (6-amino-3-chloro-2-fluorophenyl)boronic acid (5.0 g, 26.5 mmol), 4-bromo-2-methoxypyridine (5.5 g, 29.1 mmol), and potassium carbonate (7.3 g, 53 mmol) were dissolved in 1,4-dioxane (100 mL) and water (27 mL). [1,1'-Bis(diphenylphosphino)ferrocene]palladium(II) dichloride (969 mg, 1.33 mmol) was added. The system was purged with N₂ three times and then reacted at 85 °C for 16 hours.

TLC monitoring indicated completion. After cooling to room temperature, the reaction mixture was filtered, and the filtrate was concentrated. Ethyl acetate (30 mL) was added for dilution, and the mixture was extracted with ethyl acetate (2 × 30 mL). The combined organic phases were washed with saturated sodium chloride solution (2 ×30 mL), dried over anhydrous sodium sulfate, and purified by silica gel column chromatography (eluent: n-hexane/ethyl acetate = 10/1) to afford 3.8 g of 4-chloro-3-fluoro-2-(2-methoxypyridin-4-yl)aniline as a pale yellow solid (yield: 57.1%). LCMS: RT = 2.05 min, [M+H]⁺ = 253.02.

### Step B: Synthesis of 4-(6-azido-3-chloro-2-fluorophenyl)-2-methoxypyridine

At room temperature, 4-chloro-3-fluoro-2-(2-methoxypyridin-4-yl)aniline (660 mg, 2.62 mmol), tert-butyl nitrite (396 mg, 3.82 mmol), and trimethylsilyl azide (456 mg, 3.92 mmol) were dissolved in acetonitrile (14 mL). The reaction was stirred under N₂ protection at room temperature for 1 hour.

TLC monitoring indicated completion. After the reaction mixture cooled to room temperature, saturated brine (20 mL) was added dropwise to quench the reaction. The mixture was extracted with ethyl acetate (2 × 20 mL). The combined organic phases were washed with saturated brine (3 × 20 mL), dried over anhydrous sodium sulfate, and purified by silica gel column chromatography (eluent: n-hexane/ethyl acetate = 20/1) to afford 694 mg of 4-(6-azido-3-chloro-2-fluorophenyl)-2-methoxypyridine as a pale yellow solid (yield: 96%). LCMS: RT = 1.95 min, [M+H]⁺ = 279.03.

### Step C: Synthesis of 4-(3-chloro-2-fluoro-6-(4-(trifluoromethyl)-1H-1,2,3-triazol-1-yl) phenyl)-2-methoxypyridine

At room temperature, 4-(6-azido-3-chloro-2-fluorophenyl)-2-methoxypyridine (694 mg, 2.49 mmol) and copper(I) oxide (56 mg, 0.39 mmol) were dissolved in acetonitrile (14 mL). 3,3,3-Trifluoropropyne gas was bubbled into the system for 1 hour, and the reaction was stirred at room temperature for 3 hours.

TLC monitoring indicated completion. The reaction mixture was filtered, and the filtrate was concentrated. Ethyl acetate (20 mL) was added for dilution. The organic phase was washed with saturated sodium chloride solution (2 × 20 mL), dried over anhydrous sodium sulfate, and purified by silica gel column chromatography (eluent: n-hexane/ethyl acetate = 10/1) to afford 460 mg of 4-(3-chloro-2-fluoro-6-(4-(trifluoromethyl)-1*H*-1,2,3-triazol-1-yl)phenyl)-2-methoxypyridine as a pale yellow solid (yield: 49.8%). LCMS: RT = 2.09 min, [M+H]⁺ = 327.97.

### Step D: Synthesis of 4-(3-chloro-2-fluoro-6-(4-(trifluoromethyl)-1H-1,2,3-triazol-1-yl) phenyl)pyridin-2(1H)-one

At room temperature, 4-(3-chloro-2-fluoro-6-(4-(trifluoromethyl)-1*H*-1,2,3-triazol-1-yl) phenyl)-2-methoxypyridine (460 mg, 1.24 mmol) was dissolved in isopropanol (6 mL). p-Toluenesulfonic acid monohydrate (1.4 g, 7.42 mmol) and lithium chloride (736 mg, 17.4 mmol) were added. The reaction was stirred under N₂ protection at 75 °C for 16 hours.

LC-MS monitoring indicated complete consumption of the starting material. After cooling to room temperature, the reaction mixture was filtered, and the filtrate was concentrated. The residue was extracted with ethyl acetate (2 × 10 mL). The combined organic phases were washed with saturated brine (3 × 10 mL), dried over anhydrous sodium sulfate, and purified by silica gel column chromatography (eluent: pure ethyl acetate) to afford 430 mg of 4-(3-chloro-2-fluoro-6-(4-(trifluoromethyl)-1*H*-1,2,3-triazol-1-yl)phenyl)pyridin-2(1*H*)-one as a white solid (yield: 96.8%). LCMS: RT = 1.72 min, [M+H]⁺ = 359.02.

### Step E: Synthesis of (3S,7R)-3-(4-chloro-2-fluoro-6-(4-(trifluoromethyl)-1H-1,2,3-triazol-1-yl)phenyl)-2-oxopyridin-1(2H)-yl)-2⁴2⁵-difluoro-7-methyl-1¹-(methyl-d₃)-1¹H-9-az a-1(5,4)-pyrazola-2(1,3)-benzenacyclononaphan-8-one

4-(3-Chloro-2-fluoro-6-(4-(trifluoromethyl)-1*H*-1,2,3-triazol-1-yl)phenyl)pyridin-2(1*H*)-on e (70 mg, 0.19 mmol) was dissolved in anhydrous 1,4-dioxane (7 mL). Lithium bis(trimethylsilyl)amide (1 M solution in tetrahydrofuran, 0.21 mL, 0.21 mmol) was added dropwise, and the resulting reaction mixture was stirred at room temperature for 30 minutes. Then, (7*R*)-2⁴,2⁵-difluoro-7-methyl-1¹-(methyl-*d*₃)-8-oxo-1¹*H*-9-aza-1(5,4)-pyrazola-2(1,3)-benzenacyclononaphan-3-yl 4-nitrobenzenesulfonate (100 mg, 0.19 mmol) was added, and the reaction was carried out at 70 °C for 24 hours.

LC-MS monitoring indicated completion. The mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 11 mg of (3*S*,7*R*)-3-(4-chloro-2-fluoro-6-(4-(trifluoromethyl)-1*H*-1,2,3-triazol-1-yl) phenyl)-2-oxopyridin-1(2*H*)-yl)-2⁴,2⁵-difluoro-7-methyl-1¹-(methyl-*d*₃)-1¹*H*-9-aza-1(5,4)-pyrazo la-2(1,3)-benzenacyclononaphan-8-one as a white solid. LCMS: RT = 1.95 min, [M+H]⁺ = 679.07.

### Example 16

### Synthesis of (3S,7R)-3-(4-(3-chloro-2-fluoro-6-(4-(trifluoromethyl)-1H-1,2,3-triazol-1-yl)phenyl)-6-oxopyrimidin-1(6H)-yl)-2⁵2⁶-difluoro-7-methyl-1¹-(methyl-d₃)-1¹H-9-aza-1(5,4 )-pyrazola-2(1,3)-benzenacyclononaphan-8-one (16)

The specific synthetic route is as follows:

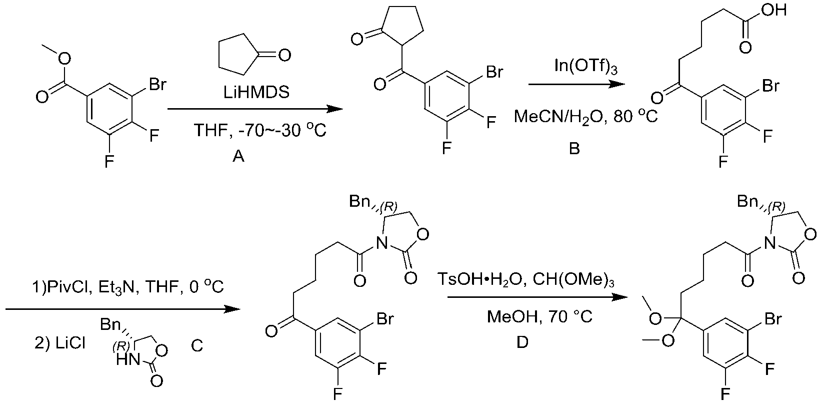

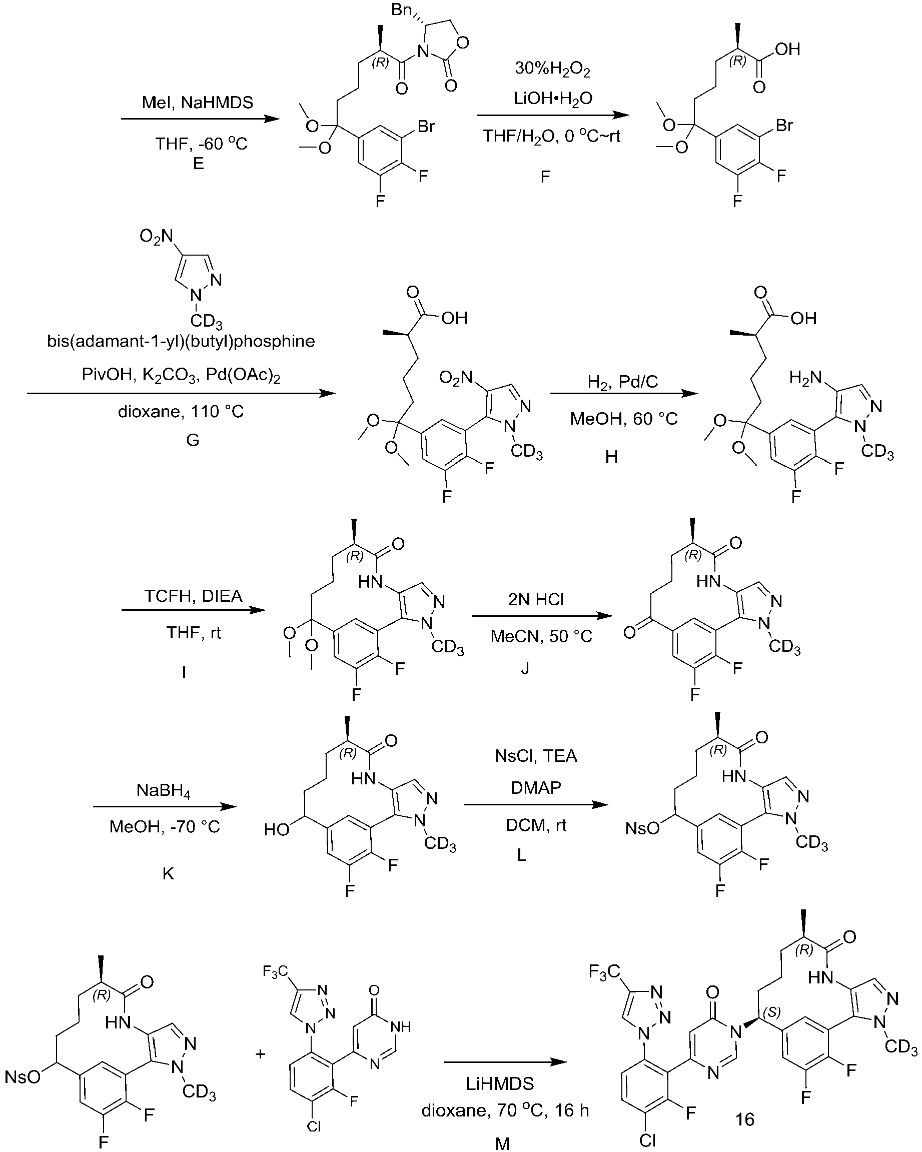

### Step A: Synthesis of 2-(3-bromo-4,5-difluorobenzoyl)cyclopentan-1-one

At room temperature, cyclopentanone (40.2 g, 478 mmol) and methyl 3-bromo-4,5-difluorobenzoate (100 g, 398 mmol) were dissolved in tetrahydrofuran (1000 mL). A solution of lithium bis(trimethylsilyl)amide in tetrahydrofuran (1 M, 419.2 mL, 419.2 mmol) was added dropwise at -70 °C. After the addition, the temperature was allowed to rise naturally to -30 °C. The reaction was then stirred at -30 °C for 16 hours.

TLC monitoring indicated completion. The reaction was quenched with 2 N hydrochloric acid solution (150 mL). After separation, most of the tetrahydrofuran was removed from the organic phase under reduced pressure. Ethyl acetate (500 mL) was added, and the mixture was washed with saturated brine (1 × 100 mL). The organic phase was dried over anhydrous sodium sulfate and concentrated under reduced pressure. The residue was purified by silica gel column chromatography (eluent: ethyl acetate/n-hexane = 1/9) to afford 64 g of 2-(3-bromo-4,5-difluorobenzoyl)cyclopentan-1-one as a pale yellow oil (yield: 53%).

### Step B: Synthesis of 6-(3-bromo-4,5-difluorophenyl)-6-oxohexanoic acid

At room temperature, 2-(3-bromo-4,5-difluorobenzoyl)cyclopentan-1-one (100 g, 0.33 mol) was added to 1200 mL of a mixture of acetonitrile and water (1:1). Indium(III) trifluoromethanesulfonate (18.6 g, 0.033 mol) was added. The mixture was heated to 80 °C and reacted for 16 hours.

TLC monitoring indicated completion. After cooling to room temperature, solid sodium chloride was added to the reaction mixture. After separation, the aqueous phase was extracted with ethyl acetate (2 × 300 mL). The combined organic phases (from three extractions) were dried and concentrated under reduced pressure. The residue was purified by silica gel column chromatography (eluent: ethyl acetate/n-hexane = 2/3) to afford 68.5 g of 6-(3-bromo-4,5-difluorophenyl)-6-oxohexanoic acid as a white solid (yield: 65%). LCMS: RT = 1.97 min, [M-H]⁻ = 318.95.

### Step C: Synthesis of (R)-1-(4-benzyl-2-oxooxazolidin-3-yl)-6-(3-bromo-4,5-difluorophenyl)hexane-1,6-dione

6-(3-Bromo-4,5-difluorophenyl)-6-oxohexanoic acid (68.5 g, 214 mmol) and triethylamine (82.2 mL, 535 mmol) were dissolved in anhydrous tetrahydrofuran (1000 mL). Pivaloyl chloride (39.4 mL, 318 mmol) was added at 0 °C, and the mixture was stirred for 0.5 hours. Lithium chloride (13.36 g, 318 mmol) was added, and stirring was continued for 15 minutes. Then, (R)-4-benzyloxazolidin-2-one (45.6 g, 257 mmol) was added in one portion, and the reaction was stirred at room temperature for 3 hours.

After monitoring indicated completion, the reaction was quenched with saturated aqueous ammonium chloride solution (500 mL). After separation, most of the tetrahydrofuran was removed from the organic phase under reduced pressure. Ethyl acetate (500 mL) was added, and the mixture was washed with saturated brine (1 × 100 mL). The organic phase was dried over anhydrous sodium sulfate and purified by silica gel column chromatography (eluent: n-hexane:ethyl acetate = 7:3) to afford 95.5 g of (*R*)-1-(4-benzyl-2-oxooxazolidin-3-yl)-6-(3-bromo-4,5-difluorophenyl)hexane-1,6-dione as a pale yellow oil (yield: 93%). LCMS: RT = 2.29 min, [M+H]⁺-CH₃O⁻⁻= 493.97.

### Step D: Synthesis of (R)-4-benzyl-3-(6-(3-bromo-4,5-difluorophenyl)-6,6-dimethoxy hexanoyl)oxazolidin-2-one

At room temperature, (*R*)-1-(4-benzyl-2-oxooxazolidin-3-yl)-6-(3-bromo-4,5-difluoro phenyl)hexane-1,6-dione (95.5 g, 199 mmol) was dissolved in methanol (955 mL). Trimethyl orthoformate (63.4 g, 597 mmol) and p-toluenesulfonic acid monohydrate (11.4 g, 59.7 mmol) were added sequentially. The reaction was stirred at 70 °C for 3 hours.

TLC indicated completion. The reaction mixture was cooled to room temperature, and the pH was adjusted to 9 with saturated sodium bicarbonate solution. Most of the methanol was removed under reduced pressure. The mixture was extracted with ethyl acetate (3 × 300 mL). The combined organic phases were washed with saturated sodium chloride solution (1 × 100 mL), dried over anhydrous sodium sulfate, and concentrated under reduced pressure. The residue was purified by silica gel column chromatography (eluent: n-hexane:ethyl acetate = 7:3) to afford 83 g of (*R*)-4-benzyl-3-(6-(3-bromo-4,5-difluorophenyl)-6,6-dimethoxyhexanoyl) oxazolidin-2-one as a pale yellow oil (yield: 79%). LCMS: RT = 2.07 min, [M+H]⁺-CH₃O⁻ = 334.94.

### Step E: Synthesis of (R)-4-benzyl-3-((R)-6-(3-bromo-4,5-difluorophenyl)-6,6-dimethoxy-2-methylhexanoyl)oxazolidin-2-one

Iodomethane (21.6 mL, 374 mmol) was added to a solution of (*R*)-4-benzyl-3-(6-(3-bromo-4,5-difluorophenyl)-6,6-dimethoxyhexanoyl)oxazolidin-2-one (83 g, 158 mmol) in tetrahydrofuran (1000 mL). The system was purged with N₂ three times, then the internal temperature was lowered to -60 °C. A solution of sodium bis(trimethylsilyl)amide in tetrahydrofuran (2 M, 118 mL) was added slowly, keeping the internal temperature below -50 °C. After the addition, the reaction was stirred at -60 °C for 1 hour.

TLC monitoring indicated completion. The reaction was quenched with saturated ammonium chloride solution (400 mL). After separation, most of the tetrahydrofuran was removed from the organic phase under reduced pressure. Ethyl acetate (600 mL) was added, and the mixture was washed with saturated brine (1 × 100 mL). The organic phase was dried over anhydrous sodium sulfate and concentrated to afford 85 g of crude (R)-4-benzyl-3-((R)-6-(3-bromo-4,5-difluorophenyl)-6,6-dimethoxy-2-methylhexanoyl)oxazolid in-2-one as a pale yellow oil, which was used directly in the next step. LCMS: RT = 2.30 min, [M+H]⁺-CH₃O⁻ = 508.17.

### Step F: Synthesis of (R)-6-(3-bromo-4,5-difluorophenyl)-6,6-dimethoxy-2-methylhexanoic acid

Crude (*R*)-4-benzyl-3-((*R*)-6-(3-bromo-4,5-difluorophenyl)-6,6-dimethoxy-2-methylhexanoyl)oxazolidin-2-one (85 g, 158 mmol) was dissolved in tetrahydrofuran (850 mL). Aqueous hydrogen peroxide solution (30 wt%, 35.7 g, 316 mmol) was added at 0 °C, and the mixture was stirred for 10 minutes. Finally, an aqueous solution of lithium hydroxide monohydrate (13.2 g, 316 mmol) in water (170 mL) was added. The reaction was stirred at room temperature for 2.0 hours.

TLC monitoring indicated completion. The reaction was quenched at 0 °C with an aqueous sodium sulfite solution (200 mL, 400 mmol) and then stirred at room temperature for 2 hours. Most of the tetrahydrofuran was removed under reduced pressure. The residue was acidified to pH ~5 with solid citric acid and then extracted with ethyl acetate (3 × 300 mL). The combined organic phases were dried over anhydrous sodium sulfate and purified by silica gel column chromatography (eluent: n-hexane:ethyl acetate = 4:1) to afford 33 g of (*R*)-6-(3-bromo-4,5-difluorophenyl)-6,6-dimethoxy-2-methylhexanoic acid as a pale yellow oil (over two steps yield: 55%). LCMS: RT = 2.11 min, [M+H]⁺-CH₃O⁻ = 348.96.

### Step G: Synthesis of (R)-6-(3,4-difluoro-5-(1-(methyl-d₃)-4-nitro-1H-pyrazol-5-yl) phenyl)-6,6-dimethoxy-2-methylhexanoic acid

(*R*)-6-(3-Bromo-4,5-difluorophenyl)-6,6-dimethoxy-2-methylhexanoic acid (25 g, 65 mmol), 1-(methyl-*d*₃)-4-nitro-1*H*-pyrazole (8.6 g, 65 mmol), potassium carbonate (22.7 g, 163 mmol), and pivalic acid (1.34 g, 13 mmol) were dissolved in anhydrous 1,4-dioxane (500 mL). Then, n-butyl di(1-adamantyl)phosphine (2.36 g, 6.5 mmol) and palladium acetate (1.47 mg, 6.5 mmol) were added. After nitrogen exchange, the reaction was stirred at 110 °C for 16 hours.

TLC monitoring indicated completion. The reaction mixture was cooled, and most of the dioxane was removed under reduced pressure. Water was added, and the pH was adjusted to ~5 with solid citric acid. The mixture was extracted with ethyl acetate (3 × 400 mL). The combined organic phases were washed with saturated sodium chloride solution (1 × 100 mL), dried over anhydrous sodium sulfate, and purified by silica gel column chromatography (eluent: n-hexane:ethyl acetate = 4:1) to afford 18 g of (*R*)-6-(3,4-difluoro-5-(1-(methyl-*d*₃)-4-nitro-1*H-*pyrazol-5-yl)phenyl)-6,6-dimethoxy-2-methylhexanoic acid as a pale yellow foamy solid (yield: 64%). LCMS: RT = 1.99 min, [M-H]⁻ = 429.08.

### Step H: Synthesis of (R)-6-(3-(4-amino-1-(methyl-d₃)-1H-pyrazol-5-yl)-4,5-difluoro phenyl)-6,6-dimethoxy-2-methylhexanoic acid

At room temperature, (*R*)-6-(3,4-difluoro-5-(1-(methyl-*d*₃)-4-nitro-1*H*-pyrazol-5-yl)phenyl) -6,6-dimethoxy-2-methylhexanoic acid (3.3 g, 7.6 mmol) was dissolved in methanol (99 mL). Pd/C (10% Pd with 50% water content, 1.32 g) was added. After hydrogen exchange, a hydrogen balloon was attached, and the reaction was stirred at 60 °C for 6 hours.

TLC monitoring indicated completion. The reaction mixture was cooled to room temperature, filtered through Celite, and the filtrate was dried and concentrated to afford 2.70 g of (*R*)-6-(3-(4-amino-1-(methyl-*d*₃)-1*H*-pyrazol-5-yl)-4,5-difluorophenyl)-6,6-dimethoxy-2-methylhexanoic acid as a pale red foamy solid (yield: 90%). LCMS: RT = 1.72 min, [M+H]⁺ = 401.13.

### Step I: Synthesis of (R)-2⁵,2⁶-difluoro-3,3-dimethoxy-7-methyl-1¹-(methyl-d₃)-1¹H -9-aza-1(5,4)-pyrazola-2(1,3)-benzenacyclononaphan-8-one

At room temperature, a solution of (*R*)-6-(3-(4-amino-1-(methyl-*d*₃)-1*H*-pyrazol-5-yl)-4,5-difluorophenyl)-6,6-dimethoxy-2-methylhexanoic acid (2.7 g, 6.75 mmol) in tetrahydrofuran (100 mL) was slowly added dropwise to a mixture of N,N,N',N'-tetramethylchloroformamidinium hexafluorophosphate (3.85 g, 13.50 mmol) and N,N-diisopropylethylamine (1.78 g, 13.50 mmol) in tetrahydrofuran (400 mL). After the addition was complete, the reaction was stirred at room temperature for 1 hour.

TLC monitoring indicated completion. The reaction mixture was diluted with water. Most of the tetrahydrofuran was removed under reduced pressure, and the mixture was extracted with ethyl acetate (3 × 50 mL). The combined organic phases were dried over anhydrous sodium sulfate and concentrated under reduced pressure to afford 5 g of crude (*R*)-2⁵,2⁶-difluoro-3,3-dimethoxy-7-methyl-1¹-(methyl-*d*₃)-1¹*H*-9-aza-1(5,4)-pyrazola-2(1,3)-ben zenacyclononaphan-8-one as a pale yellow foamy solid, which was used directly in the next step. LCMS: RT = 1.84 min, [M+H]⁺ = 383.13.

### Step J: Synthesis of (R)-2⁵,2⁶-difluoro-7-methyl-1¹-(methyl-d₃)-1¹H-9-aza-1(5,4)-pyrazola-2(1,3)-benzenacyclononaphan-3,8-dione

Crude (*R*)-2⁵,2⁶-difluoro-3,3-dimethoxy-7-methyl-1'-(methyl-*d*₃)-1¹*H*-9-aza-1(5,4)-pyrazola-2(1,3)-benzenacyclononaphan-8-one (5 g, 13 mmol) was dissolved in acetonitrile (45 mL). A dilute hydrochloric acid solution (2 M in water, 15 mL) was added, and the reaction was stirred at 50 °C for 4 hours.

TLC monitoring indicated completion. The mixture was neutralized to pH 7 with saturated sodium bicarbonate solution and extracted with ethyl acetate (2 × 30 mL). The combined organic phases were dried over anhydrous sodium sulfate, concentrated, and purified by silica gel column chromatography (eluent: n-hexane:ethyl acetate = 1:4) to afford 1.87 g of (*R*)-2⁵,2⁶-difluoro-7-methyl-1¹-(methyl-*d*₃)-1¹*H*-9-aza-1(5,4)-pyrazola-2(1,3)-benzenacyclonona phan-3,8-dione as a white solid (over two steps yield: 82%). LCMS: RT = 1.75 min, [M+H]⁺ = 337.11.

### Step K: Synthesis of (7R)-2⁵,2⁶-difluoro-3-hydroxy-7-methyl-1¹-(methyl-d₃)-1¹H-9-aza-1(5,4)-pyrazola-2(1,3)-benzenacyclononaphan-8-one

(*R*)-2⁵,2⁶-difluoro-7-methyl-1¹-(methyl-*d*₃)-1¹*H*-9-aza-1(5,4)-pyrazola-2(1,3)-benzenacyclo nonaphan-3,8-dione (1.87 g, 5.56 mmol) was dissolved in anhydrous methanol (36 mL). Sodium borohydride (211 mg, 5.56 mmol) was added in portions at -70 °C, and the reaction was stirred at this temperature for 15 minutes.

TLC monitoring indicated completion. The reaction was quenched with saturated ammonium chloride solution and extracted with ethyl acetate (5 × 30 mL). The combined organic phases were dried over anhydrous sodium sulfate and concentrated to afford 900 mg of (7*R*)-2⁵,2⁶-difluoro-3-hydroxy-7-methyl-1¹-(methyl-*d*₃)-1¹*H*-9-aza-1(5,4)-pyrazola-2(1,3)-benzen acyclononaphan-8-one as a white solid (yield: 48%). LCMS: RT = 1.62 min, [M+H]⁺ = 339.04.

### Step L: Synthesis of (7R)-2⁵,2⁶-difluoro-7-methyl-1¹-(methyl-d₃)-8-oxo-1¹H-9-aza-1(5,4)-pyrazola-2(1,3)-benzenacyclononaphane-3-yl 4-nitrobenzenesulfonate

(7*R*)-2⁵,2⁶-difluoro-3-hydroxy-7-methyl-1¹-(methyl-*d*₃)-1¹*H*-9-aza-1(5,4)-pyrazola-2(1,3)-b enzenacyclononaphan-8-one (900 mg, 2.66 mmol) was dissolved in anhydrous dichloromethane (50 mL). Triethylamine (672 mg, 6.66 mmol) and 4-dimethylaminopyridine (324 mg, 2.66 mmol) were added sequentially, and the mixture was stirred for 10 minutes. Then, 4-nitrobenzenesulfonyl chloride (882 mg, 3.99 mmol) was added in portions, and the reaction was stirred at room temperature for 4 hours.

TLC monitoring indicated completion. The mixture was diluted with sodium bicarbonate solution (30 mL) and extracted with dichloromethane (2 × 20 mL). The combined organic phases were dried over anhydrous sodium sulfate and concentrated under reduced pressure. The residue was purified by column chromatography (eluent: n-hexane:ethyl acetate = 1:15) to afford 900 mg of (7*R*)-2⁵,2⁶-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate as a pale yellow solid (yield: 65%). LCMS: RT = 1.93 min, [M+H]⁺ = 524.02.

### Step M: Synthesis of (3S,7R)-3-(4-(3-chloro-2-fluoro-6-(4-(trifluoromethyl)-1H-1,2,3-triazol-1-yl)phenyl)-6-oxopyrimidin-1(6H)-yl)-2⁵,2⁶-difluoro-7-methyl-1¹-(methyl-d₃)-1¹H-9-aza-1(5,4)-pyrazola-2(1,3)-benzenacyclononaphan-8-one

6-(3-Chloro-2-fluoro-6-(4-(trifluoromethyl)-1*H*-1,2,3-triazol-1-yl)phenyl)pyrimidin-4(3*H*)-one (160 mg, 0.44 mmol) was dissolved in dry 1,4-dioxane (10 mL). Lithium bis(trimethylsilyl)amide (1 M solution in tetrahydrofuran, 0.44 mL, 0.44 mmol) was added dropwise, and the resulting reaction mixture was stirred at room temperature for 30 minutes. Then, (7*R*)-2⁵,2⁶-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (230 mg, 0.44 mmol) was added, and the reaction was carried out at 70 °C for 16 hours.

TLC monitoring indicated completion. The mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 45 mg of (3*S*,7*R*)-3-(4-(3-chloro-2-fluoro-6-(4-(trifluoromethyl)-1*H*-1,2,3-triazol-1-yl) phenyl)-6-oxopyrimidin-1(6*H*)-yl)-2⁵2⁶-difluoro-7-methyl-1¹-(methyl-*d*₃)-1¹*H*-9-aza-1(5,4)-pyrazola-2(1,3)-benzenacyclononaphan-8-one as a white solid (yield: 15%). LC-MS: RT = 2.00 min, [M+H]⁺ =679.99. ¹H NMR (400 MHz, DMSO-*d*₆) δ 9.20 (s, 1H), 9.19 (s, 1H), 8.47 (brs, 1H), 8.05 (t, *J* = 8.2 Hz, 1H), 7.77 (dd, *J* = 8.7, 1.4 Hz, 1H), 7.51 (s, 1H), 7.42-7.37 (m, 2H), 6.71 (s, 1H), 5.54 (d, *J =* 12.6 Hz, 1H), 2.56-2.52 (m, 1H), 2.47-2.39 (m, 1H), 2.39-2.28 (m, 1H), 1.90-1.83 (m, 1H), 1.82-1.72(m, 1H), 1.45-1.33(m, 1H), 1.14-1.04(m, 1H), 0.95 (d, *J* = 6.7 Hz, 3H).

### Example 17

### Synthesis of (3S,7R)-3-(4-(5-chloro-2-(4-chloro-1H-1,2,3-triazol-1-yl)phenyl)-6-oxopyrimidin-1(6H)-yl)-2⁵,2⁶-difluoro-7-methyl-1¹-(methyl-d₃)-1¹H-9-aza-1(5,4)-pyrazola-2( 1,3)-benzenacyclononaphan-8-one (17)

The specific synthetic route is as follows:

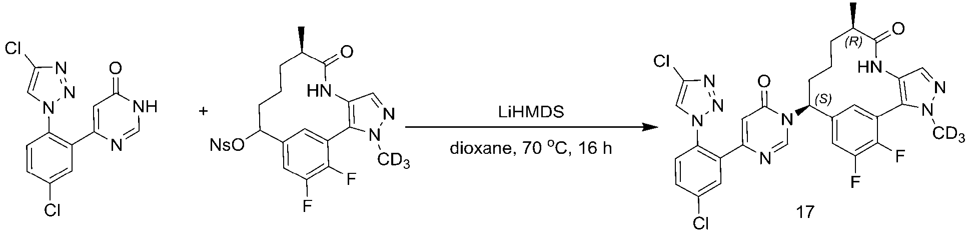

6-(5-Chloro-2-(4-chloro-1*H*-1,2,3-triazol-1-yl)phenyl)pyrimidin-4(3*H*)-one (100 mg, 0.325 mmol) was dissolved in dry 1,4-dioxane (10 mL). Lithium bis(trimethylsilyl)amide (1 M solution in tetrahydrofuran, 0.325 mL, 0.325 mmol) was added dropwise, and the resulting reaction mixture was stirred at room temperature for 30 minutes. Then, (7*R*)-2⁵,2⁶-difluoro-7-methyl-1¹-(methyl-*d*₃)-8-oxo-1¹*H*-9-aza-1(5,4)-pyrazola-2(1,3)-benzenacyc lononaphane-3-yl 4-nitrobenzenesulfonate (170 mg, 0.325 mmol) was added, and the reaction was carried out at 70 °C for 16 hours.

TLC monitoring indicated completion. The mixture was cooled to room temperature. Saturated aqueous ammonium chloride solution (25 mL) and ethyl acetate (50 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 19.7 mg of (3*S*,7*R*)-3-(4-(5-chloro-2-(4-chloro-1*H*-1,2,3-triazol-1-yl)phenyl)-6-oxopyrimidin-1(6*H*)-yl)-2⁵,2⁶ -difluoro-7-methyl-1¹-(methyl-*d*₃)-1¹*H*-9-aza-1(5,4)-pyrazola-2(1,3 )-benzenacyclononaphan-8-one as a white solid (yield: 9.7 LC-MS: RT = 1.95 min, [M+H]⁺ =627.98.

¹H NMR (400 MHz, DMSO-*d*₆) δ 9.20 (s, 1H), 8.74 (s, 1H), 8.48 (s, 1H), 7.89 (d, J = 2.3 Hz, 1H), 7.82 (dd, J = 8.5, 2.4 Hz, 1H), 7.75 (d, J = 8.5 Hz, 1H), 7.54 - 7.45 (m, 2H), 7.45 - 7.37 (m, 1H), 6.40 (s, 1H), 5.52 (d, J = 12.6 Hz, 1H), 2.47 - 2.30 (m, 2H), 1.95 - 1.82 (m, 1H), 1.82 - 1.69 (m, 1H), 1.45 - 1.33 (m, 1H), 1.17 - 1.02 (m, 2H), 0.94 (d, J = 6.7 Hz, 3H).

### Example 18

### Synthesis of (3S,7R)-3-(4-(5-chloro-2-(4-(trifluoromethyl)-1H-1,2,3-triazol-1-yl)phenyl) -6-oxopyrimidin-1(6H)-yl)-2⁵,2⁶-difluoro-7-methyl-1¹-(methyl-d₃)-1¹H-9-aza-1(5,4)-pyrazola -2(1,3)-benzenacyclononaphan-8-one (18)

The specific synthetic route is as follows:

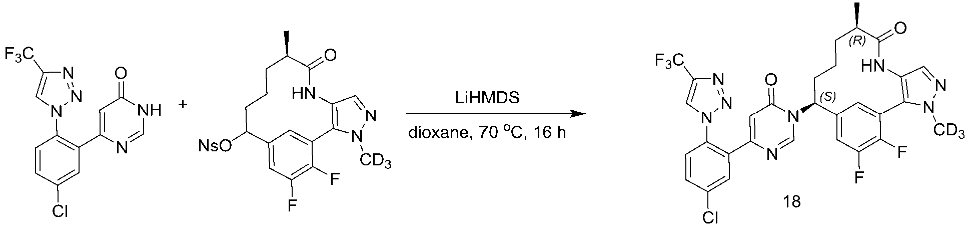

6-(5-Chloro-2-(4-(trifluoromethyl)-1*H*-1,2,3-triazol-1-yl)phenyl)pyrimidin-4(3*H*)-one (200.0 mg, 0.59 mmol) was dissolved in dry 1,4-dioxane (10 mL). Lithium bis(trimethylsilyl)amide (1 M solution in tetrahydrofuran, 0.59 mL, 0.59 mmol) was added dropwise, and the resulting reaction mixture was stirred at room temperature for 30 minutes. Then, (7*R*)-2⁵,2⁶-difluoro-7-methyl-1¹-(methyl-*d*₃)-8-oxo-1¹*H*-9-aza-1(5,4)-pyrazola-2(1,3)-benzenacyclononaphane-3-yl 4-nitrobenzenesulfonate (307 mg, 0.59 mmol) was added, and the reaction was carried out at 70 °C for 16 hours.

TLC monitoring indicated completion. The mixture was cooled to room temperature. Saturated aqueous ammonium chloride solution (25 mL) and ethyl acetate (30 mL) were added. The mixture was separated, and the organic phase was washed with saturated brine. The organic phase was dried over anhydrous sodium sulfate and concentrated under reduced pressure. The residue was purified by silica gel column chromatography (eluent: ethyl acetate/petroleum ether = 14/1), followed by purification using preparative high-performance liquid chromatography to afford 40.1 mg of (3*S*,7*R*)-3-(4-(5-chloro-2-(4-(trifluoromethyl)-1*H*-1,2,3-triazol-1-yl)phenyl)-6-oxopyrimidin-1(6*H*)-yl)-2⁵,2⁶-difluoro-7-methyl-1¹-(methyl-*d*₃)-1¹*H*-9-aza-1(5,4)-pyrazola-2(1,3 )-benzenacyclononaphan-8-one as a white solid (yield: 10.2%). LC-MS: RT = 1.98 min, [M+H]⁺ = 662.00.

¹H NMR (400 MHz, DMSO-d6) δ 9.26 - 9.14 (m, 2H), 8.51 - 8.36 (m, 1H), 7.93 (d, 1H), 7.89 - 7.79 (m, 2H), 7.51 (s, 1H), 7.48 - 7.36 (m, 2H), 6.52 (s, 1H), 5.53 (d, J = 12.4 Hz, 1H), 3.31 (s, 1H), 2.47 - 2.36 (m, 2H), 1.92 - 1.81 (m, 1H), 1.81 - 1.69 (m, 1H), 1.46 - 1.33 (m, 1H), 1.18 - 1.02 (m, 1H), 0.95 (d, J = 6.7 Hz, 3H).

### Example 19

### Synthesis of (3S,7R)-3-(4-(3-chloro-6-(4-chloro-1H-1,2,3-triazol-1-yl)-2-fluorophenyl)-6-oxopyrimidin-1(6H)-yl)-2⁵,2⁶-difluoro-7-methyl-1¹-(methyl-d₃)-1¹H-9-aza-1(5,4)-pyrazola-2(1,3)-benzenacyclononaphan-8-one (19)

The specific synthetic route is as follows:

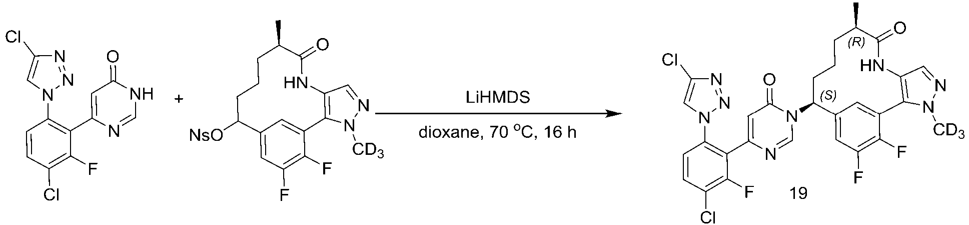

6-(3-Chloro-6-(4-chloro-1*H*-1,2,3-triazol-1-yl)-2-fluorophenyl)pyrimidin-4(3*H*)-one (70 mg, 0.215 mmol) was dissolved in dry 1,4-dioxane (7 mL). Lithium bis(trimethylsilyl)amide (1 M solution in tetrahydrofuran, 0.215 mL, 0.215 mmol) was added dropwise, and the resulting reaction mixture was stirred at room temperature for 30 minutes. Then, (7*R*)-2⁵,2⁶-difluoro-7-methyl-1¹-(methyl-*d*₃)-8-oxo-1¹*H*-9-aza-1(5,4)-pyrazola-2(1,3)-benzenacyc lononaphan-3-yl 4-nitrobenzenesulfonate (170 mg, 0.323 mmol) was added, and the reaction mixture was stirred at 70 °C for 16 hours.

After TLC monitoring indicated completion, the mixture was cooled to room temperature. Saturated aqueous ammonium chloride solution (25 mL) and ethyl acetate (30 mL) were added. The mixture was separated, and the organic phase was washed with saturated brine, dried over anhydrous sodium sulfate, and concentrated under reduced pressure. The residue was purified by silica gel column chromatography (eluent: ethyl acetate/petroleum ether = 10/1), followed by purification using preparative high-performance liquid chromatography to afford 17.6 mg of (3*S*,7*R*)-3-(4-(3-chloro-6-(4-chloro-1*H*-1,2,3-triazol-1-yl)-2-fluorophenyl)-6-oxopyrimidin-1(6*H* )-yl)-2⁵,2⁶-difluoro-7-methyl-1¹-(methyl-*d*₃)-1¹H-9-aza-1(5,4)-pyrazola-2(1,3)-benzenacyclonon aphan-8-one as a white solid (yield: 12.7%). LC-MS: RT = 1.95 min, [M+H]⁺ =646.01.

### Examples 20-23

According to the preparation methods described in the preceding examples, the following compounds 20-23 were prepared:

| | | | |
|---|---|---|---|
| 20 | 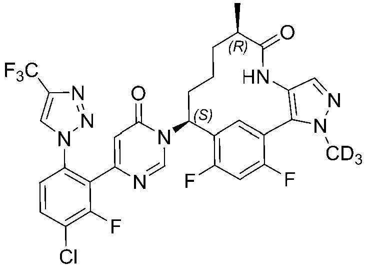 [M+H]⁺ = 681.03 | 21 | 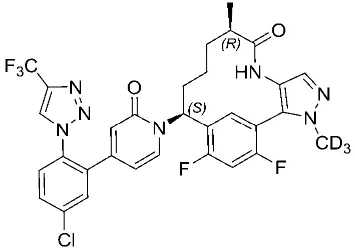 [M+H]⁺ = 662.05 |
| 22 | 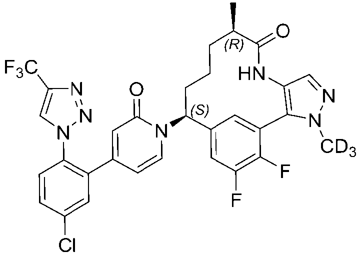 [M+H]⁺ = 662.05 | 23 | 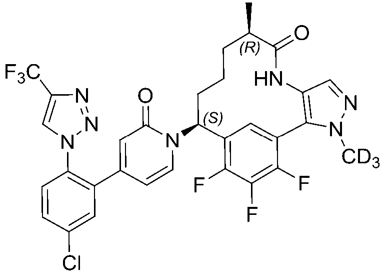 [M+H]⁺ = 680.04 |

### Example 24: Detection of the Biological Activity of the Compounds of the Present Application in Inhibiting Human Coagulation Factor XIa by Absorbance Method

### 1. Experimental Materials

Enzyme: Human Factor XIa (ENZYME RESEARCH, Cat. No. HFXIa 1111a)
Substrate: S-2366^{™} (CHROMOGENIX, Cat. No. 82109039)
Buffer: 145 mM NaCl, 5 mM KCl, 1 mg/mL PEG 8000, 30 mM HEPES, pH 7.4.

### 2. Experimental Procedure

Test compounds dissolved in 100% DMSO at 10 mM were diluted with 100% DMSO to concentrations of 1000 µM, 200 µM, 40 µM, 8 µM, 1.6 µM, 0.32 µM, 0.064 µM, 0.0128 µM, 0.00256 µM, and 0.00128 µM. Then 98 µL of FXIa enzyme solution (77.7 ng/mL) was added to each well of a 96-well plate, with 98 µL of buffer added to blank wells instead. Then, 2 µL of compound at different concentrations was added; DMSO was used instead for blank and control wells. The plate was mixed using a plate shaker and incubated at 37 °C for 20 minutes.

Finally, 100 µL of substrate (800 µM) was added to each well, and the absorbance was measured at 405 nm.

### 3. Data Processing

Curve fitting was performed using GraphPad Prism software to calculate the IC₅₀ values.

### Example 25: Determination of the In Vitro Anticoagulant Effect of the Compounds of the Present Application in Human Plasma

### 1. Experimental Materials

aPTT (Lot 220106600), Calcium Chloride (Lot 210305600), Needle Rinse Solution (Lot 211101300): produced by Shenzhen Mindray Bio-Medical Electronics Co., Ltd. Pooled human plasma: collected from healthy individuals using disposable venous blood collection tubes (Liuyang Sanli Medical Technology Development Co., Ltd., Lot 211108, sodium citrate 9:1). Blood was collected, centrifuged at room temperature at 3000 rpm for 10 minutes, and the supernatant was collected and stored for use (Lot 20220606).

### 2. Experimental Procedure

Test compounds dissolved in 100% DMSO at 10 mM were diluted with 100% DMSO to concentrations of 0.29 µM, 0.59 µM, 1.17 µM, 2.34 µM, 4.69 µM, 9.38 µM, 18.75 µM, and 37.50 µM. Then 99 µL of pooled human plasma was added to each 1.5 mL centrifuge tube, followed by the addition of 1 µL of compound at different concentrations; 1 µL of DMSO was added to the blank control. The tubes were mixed by repeated manual inversion, centrifuged briefly using a mini centrifuge to collect the samples, and then incubated in a thermostatic water bath at 37 °C for 10 minutes with mixing. The aPTT was measured using a fully automated coagulation analyzer according to the preset program.

### 3. Data Processing

Curve fitting was performed using GraphPad Prism software to calculate EC1.5x.

The results of Examples 24 and 25 are shown in Table 1:

**Table 1:**

| Compound No. | Recombinant Enzyme FXIa IC₅₀ (nM) | Human Plasma APTT (µM) |
|---|---|---|
| | | 1.5x |
| 1 | 0.24 | / |
| 2 | 0.36 | 0.24 |
| 3 | 0.61 | 0.24 |
| 4 | 0.62 | 0.20 |
| 5 | 0.89 | 0.83 |
| 6 | 1.06 | 0.43 |
| 7 | 0.34 | 0.22 |
| 8 | 0.15 | / |
| 9 | 0.30 | / |
| 10 | 0.77 | 0.20 |
| 11 | 0.29 | 0.23 |
| 12 | 0.31 | 0.28 |
| 13 | 0.40 | / |
| 14 | 0.38 | / |
| 16 | 0.40 | 0.31 |
| 17 | 0.36 | 0.04 |

Conclusion: The compounds of the present application exhibit significant inhibitory activity against human FXIa and demonstrate significant anticoagulant effects in human plasma.

### Example 26: Pharmacokinetic Experiment

### 1. Reagents and Instruments

Polyethylene glycol 400 (PEG-400, Lot GORKREUT, Shaen Chemical Technology (Shanghai) Co., Ltd.), DMSO (Lot 20200319, Guangdong Guanghua Sci-Tech Co., Ltd.), normal saline (Lot C20052604, Jiangxi Kelun Pharmaceutical Co., Ltd.). LC-MS instrument (Thermo Fisher Ultimate 3000 UPLC, TSQ QUANTUM ULTRA triple quadrupole mass spectrometer, AB SCIEX 5500 QTARP).

### 2. Experimental Animals

SD rats: Male, 180-250 g, purchased from Guangdong Vital River Laboratory Animal Technology Co., Ltd.

### 3. Formulation Preparation

The test compound powder was accurately weighed, completely dissolved in DMSO, then PEG-400 was added. The mixture was vortexed and sonicated to mix, followed by the addition of normal saline. The mixture was vortexed and sonicated again to achieve a final concentration of 0.5 mg/mL (DMSO:PEG-400:NS = 5:60:35, V/V/V). The dosing volume was 10 mL/kg for oral gavage and 0.5 mL/kg for intravenous administration.

### 4. Blood Sample Collection

After intravenous or oral administration to rats, 200 µL of venous blood was collected at 5 min (not collected for oral group), 15 min, 30 min, 1 h, 2 h, 5 h, 7 h, and 24 h into heparinized EP tubes. The blood was centrifuged at 12,000 rpm for 2 minutes, and the plasma was collected and stored at -80 °C until analysis.

### 5. Bioanalysis

A precise amount of the test compound was weighed and dissolved in DMSO to 1 mg/mL as a stock solution. An appropriate volume of the compound stock solution was accurately pipetted and diluted with acetonitrile to prepare standard series solutions. 20 µL of each standard series solution was accurately pipetted and added to 180 µL of blank plasma, vortexed to mix, to prepare plasma samples equivalent to plasma concentrations of 1 ng/mL, 3 ng/mL, 10 ng/mL, 30 ng/mL, 100 ng/mL, 300 ng/mL, 1000 ng/mL, 3000 ng/mL, and 5000 ng/mL. Each concentration was analyzed in duplicate to establish the standard curve. 20 µL of plasma sample was taken, and 200 µL of an internal standard (propranolol, 5 ng/mL) in acetonitrile solution was added. The mixture was vortexed and centrifuged at 4000 rpm for 5 minutes. The supernatant was taken for LC-MS analysis. The LC-MS detection conditions were as follows:
Chromatographic Column: Waters ACQUITY^{™} PREMIER HSS T3, 50*2.1 mm, 1.8 µm.

Mobile phase A: water (0.1% formic acid), mobile phase B: acetonitrile, flow rate: 0.5 mL/min, gradient elution: see Table 2 below.

**Table 2**

| Time (min) | A(%) | B(%) |
|---|---|---|
| 0 | 95% | 5% |
| 1 | 10% | 90% |
| 2.5 | 5% | 95% |
| 2.51 | 95% | 5% |
| 3.0 | 95% | 5% |

### 6. Data Processing

After determining the plasma drug concentration by LC-MS, the pharmacokinetic parameters following administration to rats were calculated using the non-compartmental model in WinNonlin 6.1 software. The results are shown in Table 3 below.

**Table 3: Rat Pharmacokinetic Parameters of the Compounds of the Present Application (Following iv and PO Administration)**

| Compound No. | Route of Administration and Dose (mg/kg) | F% |
|---|---|---|
| 3 | iv, 1 | / |
| | PO,5 | 15.7 |
| 4 | iv, 1 | / |
| | PO,5 | 19.0 |
| 6 | iv, 1 | / |
| | PO,5 | 14.1 |
| 11 | iv, 1 | / |
| | PO,5 | 21.6 |
| 12 | iv, 1 | / |
| | PO,5 | 29.4 |
| 16 | iv, 1 | / |
| | PO,5 | 44.5 |
| 17 | iv, 1 | / |
| | PO,5 | 59.6 |
| 18 | iv, 1 | / |
| | PO,5 | 21.9 |

From the experimental results in Table 3, it can be seen that the preferred compounds of the present application exhibit good oral absorption, high absolute bioavailability, and high exposure, which are superior to the comparative compound.

### Example 27: Detection of the Biological Activity of the Compounds of the Present Application in Inhibiting Human Coagulation Factor Chymotrypsin by Absorbance Method

### 1. Experimental Materials

Enzyme: α-Chymotrypsin (Sigma, Cat. No. C8946)
Substrate: S-2586^{™} (Boatman Biotech, Cat. No. B2586)
Buffer: 0.05 M HEPES; 0.145 M NaCl; 5 mM KCl and 0.1% PEG 8000, pH 7.4

### 2. Experimental Procedure

Test compounds dissolved in 100% DMSO at 10 mM were diluted with 100% DMSO to concentrations of 100 µM, 33.33 µM, 11.11 µM, 3.704 µM, 1.235 µM, 0.412 µM, 0.137 µM, and 0.046 µM. Then 29.4 µL of Chymotrypsin enzyme solution (20 nM) was added to each well of a 384-well plate, with 29.4 µL of buffer added to blank wells instead. Then, 0.6 µL of compound at different concentrations was added; DMSO was used instead for blank and control wells. The plate was mixed using a plate shaker and incubated at 37 °C for 30 minutes.

Finally, 30 µL of substrate at a concentration of 200 µM was added to each well, and the absorbance was measured at 405 nm.

### 3. Data Processing

Curve fitting was performed using GraphPad Prism software to calculate the IC₅₀ values, as shown in Table 4.

**Table 4. Inhibitory Effect of Compounds on Chymotrypsin**

| Compound No. | IC₅₀ (nM) |
|---|---|
| 1 | 411.2 |
| 4 | 173.0 |
| 16 | 163.6 |
| 17 | 390.5 |
| 18 | 222.6 |
| Comparative compound 2 | 79.2 |
| Comparative compound 3 | 102.8 |

Experimental Conclusion: The compounds of the present application exhibit relatively weak inhibitory activity against Chymotrypsin, suggesting superior selectivity for the target over Chymotrypsin.

### Example 28: Detection of the Biological Activity of the Compounds of the Present Application in Inhibiting Human Coagulation Factor Trypsin by Absorbance Method

### 1. Experimental Materials

Enzyme: Human Trypsin (Sigma, Cat. No. T6424)
Substrate: S-2222^{™} (CHROMOGENIX, Cat. No. 82031639)
Buffer: 0.1 M sodium phosphate; 0.2 M NaCl and 0.5% PEG 8000, pH 7.4

### 2. Experimental Procedure

Test compounds dissolved in 100% DMSO at 10 mM were diluted with 100% DMSO to concentrations of 10000 µM, 3333 µM, 1111 µM, 370.4 µM, 123.5 µM, 41.2 µM, 13.7 µM, and 4.6 µM. Then, 29.4 µL of Trypsin enzyme solution (0.8 nM) was added to each well of a 384-well plate, with 29.4 µL of buffer added to blank wells instead. Then, 0.6 µL of compound at different concentrations was added; DMSO was used instead for blank and control wells. The plate was mixed using a plate shaker and incubated at 37 °C for 30 minutes.

Finally, 30 µL of substrate at a concentration of 40 µM was added to each well, and the absorbance was measured at 405 nm.

### 3. Data Processing

Curve fitting was performed using GraphPad Prism software to calculate the IC₅₀ values, as shown in Table 5.

**Table 5. Inhibitory Effect of Compounds on Trypsin**

| Compound No. | IC₅₀ (nM) |
|---|---|
| 1 | 225.4 |
| 4 | 355.2 |
| 16 | 219.2 |
| 17 | 321.9 |
| 18 | 627.1 |
| Comparative compound 2 | 83.1 |
| Comparative compound 3 | 109.6 |

Experimental Conclusion: The compounds of the present application exhibit relatively weak inhibitory activity against Trypsin, suggesting superior selectivity for the target over Trypsin.

### Example 29: Detection of the Biological Activity of the Compounds of the Present Application in Inhibiting Human Coagulation Factor FIIa by Absorbance Method

### 1. Experimental Materials

Enzyme: Human FIIa (Enzyme Research Laboratories, Cat. No. HT 1002a)
Substrate: S-2366^{™} (CHROMOGENIX, Cat. No. 82109039)
Buffer: 0.1 M sodium phosphate; 0.2 M NaCl and 0.5% PEG 8000, pH 7.4

### 2. Experimental Procedure

Test compounds dissolved in 100% DMSO at 10 mM were diluted with 100% DMSO to concentrations of 10000 µM, 3333 µM, 1111 µM, 370.4 µM, 123.5 µM, 41.2 µM, 13.7 µM, and 4.6 µM. Then 29.4 µL of FIIa enzyme solution (4 nM) was added to each well of a 384-well plate, with 29.4 µL of buffer added to blank wells instead. Then, 0.6 µL of compound at different concentrations was added; DMSO was used instead for blank and control wells. The plate was mixed using a plate shaker and incubated at 37 °C for 30 minutes.

Finally, 30 µL of substrate at a concentration of 200 µM was added to each well, and the absorbance was measured at 405 nm.

### 3. Data Processing

Curve fitting was performed using GraphPad Prism software to calculate the IC₅₀ values, as shown in Table 6.

**Table 6. Inhibitory Effect of Compounds on Coagulation Factor FIIa**

| Compound No. | IC₅₀ (nM) |
|---|---|
| 1 | 5627 |
| 16 | 9209 |
| 17 | 6920 |
| 18 | 11137 |
| Comparative compound 1 | 3520 |
| Comparative compound 2 | 3143 |
| Comparative compound 3 | 2146 |

Experimental Conclusion: The compounds of the present application exhibit relatively weak inhibitory activity against coagulation factor FIIa, suggesting superior selectivity for the target over coagulation factor FIIa.

The structural formulas of the comparative compounds are as follows:

| Comparative compound 1 | Comparative compound 2 | Comparative compound 3 |
|---|---|---|
| 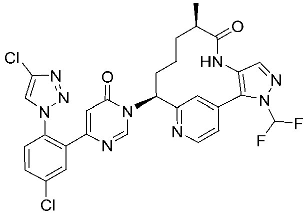 Identification data refers to references | 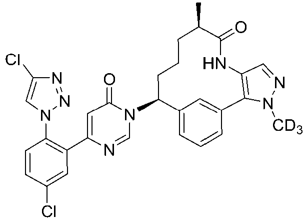 LCMS: RT = 1.84 min, [M+H]⁺ = 592.25; ¹H NMR (400 MHz, DMSO-*d*₆) δ 9.16 (s, 1H), 8.74 (s, 1H), 8.42 (s, 1H), 7.91 (d, *J* = 2.4 Hz, 1H), 7.83 (dd, *J =* 8.5, 2.4 Hz, 1H), 7.76 (d, *J =* 8.4 Hz, 1H), 7.69 (s, 1H), 7.59 - 7.51 (m, 2H), 7.43 (s, 1H), 7.24 - 7.16 (m, 1H), 6.41 (s, 1H), 5.67 - 5.57 (m, 1H), 2.43 - 2.33 (m, 1H), 1.93 - 1.81 (m, 2H), 1.51 - 1.36 (m, 1H), 1.25 - 1.07 (m, 2H), 0.98 (d, *J* = 6.8 Hz, 3H). | 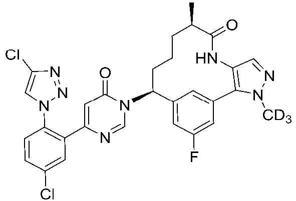 LCMS: RT = 1.93 min, [M+H] ⁺ = 609.25; ¹H NMR (400 MHz, DMSO-*d*₆) δ 9.20 (s, 1H), 8.75 (s, 1H), 8.52 (s, 1H), 7.92 (d, *J =* 2.4 Hz, 1H), 7.84 (dd, *J =* 8.5, 2.4 Hz, 1H), 7.77 (d, *J* = 8.5 Hz, 1H), 7.54 - 7.48 (m, 2H), 7.45 (s, 1H), 7.12 - 7.06 (m, 1H), 6.43 (s, 1H), 5.68 - 5.56 (m, 1H), 2.46 - 2.33 (m, 1H), 1.96 - 1.79 (m, 2H), 1.51 - 1.39 (m, 1H), 1.28 - 1.18 (m, 1H), 1.06 - 1.00 (m, 1H), 0.96 (d, *J =* 6.8 Hz, 3H). |

The aforementioned examples are preferred embodiments of the present application. However, the embodiments of the present application are not limited by the aforementioned examples. Any other alterations, modifications, substitutions, combinations, or simplifications made without departing from the spirit and principle of the present application should be considered as equivalent substitution methods and are included within the protection scope of the present application.

## Claims

1. A compound represented by general formula (I), or an isomer thereof, or a racemate thereof, or a pharmaceutically acceptable salt thereof, wherein: 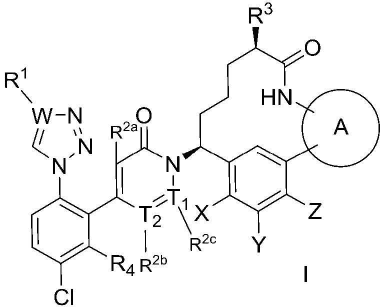
X is selected from the group consisting of hydrogen, halogen, and cyano; Y is selected from the group consisting of hydrogen, halogen, and cyano; Z is selected from the group consisting of hydrogen, halogen, and cyano; and at least two of X, Y, and Z are not hydrogen;
W is selected from the group consisting of C and N, wherein when W is N, R¹ is absent;
T₁ and T₂ are selected from the group consisting of C and N, wherein when T₁ is N, R^{2c} is absent, when T₂ is N, R^{2b} is absent, and T₁ and T₂ are not both N;
ring A is selected from the group consisting of a substituted or unsubstituted benzene ring and a substituted or unsubstituted pyrazole ring, wherein a substituent is selected from the group consisting of halogen, cyano, alkyl, and haloalkyl;
R¹ is selected from the group consisting of halogen, haloalkyl, and cyano;
R^{2a}, R^{2b}, and R^{2c} are each independently selected from the group consisting of hydrogen, halogen, and alkoxy;
R³ is selected from the group consisting of alkyl, haloalkyl, alkoxy, -(CH₂)ₙ-cycloalkyl, and -(CH₂)ₙ-heterocycloalkyl, wherein n is 0 or 1; and
R⁴ is selected from the group consisting of hydrogen and halogen.

2. The compound, or the isomer thereof, or the racemate thereof, or the pharmaceutically acceptable salt thereof according to claim 1, wherein the compound is selected from the group consisting of a compound represented by formula (Ia) or formula (Ib), or an isomer thereof, or a racemate thereof, or a pharmaceutically acceptable salt thereof: 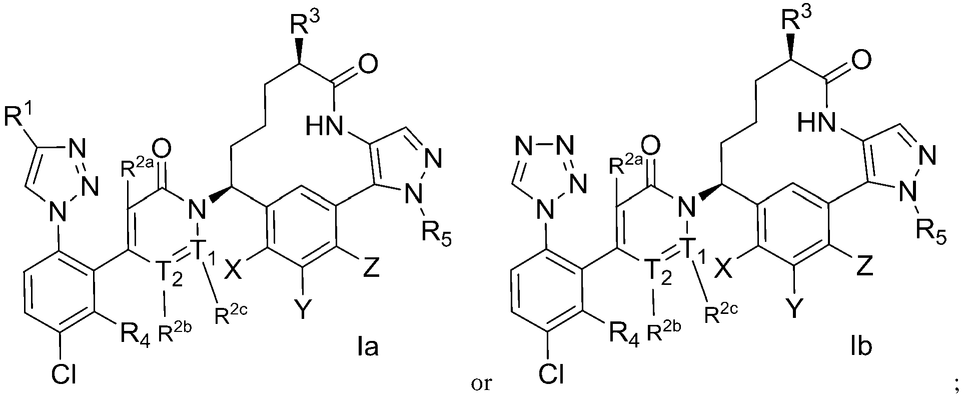
X is selected from the group consisting of hydrogen, halogen, and cyano; Y is selected from the group consisting of hydrogen, halogen, and cyano; Z is selected from the group consisting of hydrogen, halogen, and cyano; and at least two of X, Y, and Z are not hydrogen;
T₁ and T₂ are selected from the group consisting of C and N, wherein when T₁ is N, R^{2c} is absent, when T₂ is N, R^{2b} is absent, and T₁ and T₂ are not both N;
R¹ is selected from the group consisting of halogen, haloalkyl, and cyano;
R^{2a}, R^{2b}, and R^{2c} are each independently selected from the group consisting of hydrogen, halogen, and alkoxy;
R³ is selected from the group consisting of alkyl, haloalkyl, alkoxy, -(CH₂)ₙ-cycloalkyl, and -(CH₂)ₙ-heterocycloalkyl, wherein n is 0 or 1;
R⁴ is selected from the group consisting of hydrogen and halogen; and
R⁵ is selected from the group consisting of alkyl and haloalkyl.

3. The compound, or the isomer thereof, or the racemate thereof, or the pharmaceutically acceptable salt thereof according to claim 1 or 2, wherein the alkyl is C₁₋₆ alkyl, the C₁₋₆ alkyl being selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl; and
the alkoxy is C₁₋₆ alkoxy, the C₁₋₆ alkoxy being selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexyloxy, isohexyloxy, sec-hexyloxy, tert-hexyloxy, neohexyloxy, 2-methylpentyloxy, 1,2-dimethylbutoxy, and 1-ethylbutoxy.

4. The compound, or the isomer thereof, or the racemate thereof, or the pharmaceutically acceptable salt thereof according to claim 1 or 2, wherein the halogen is selected from the group consisting of fluorine, chlorine, bromine, and iodine.

5. The compound, or the isomer thereof, or the racemate thereof, or the pharmaceutically acceptable salt thereof according to claim 1 or 2, wherein the haloalkyl is an alkyl in which one or more hydrogens are replaced by halogen, the halogen being selected from the group consisting of fluorine, chlorine, bromine, and iodine.

6. The compound, or the isomer thereof, or the racemate thereof, or the pharmaceutically acceptable salt thereof according to claim 1 or 2, wherein the cycloalkyl is C₃₋₆ cycloalkyl, the C₃₋₆ cycloalkyl being selected from the group consisting of cyclopropyl, cyclobutyl, cyclopentyl, and cyclohexyl; and the heterocycloalkyl is a cycloalkyl in which one or more carbon atoms are replaced by a heteroatom, the heteroatom being selected from the group consisting of nitrogen, oxygen, and sulfur, and a number of the heteroatom being one or more.

7. The compound, or the isomer thereof, or the racemate thereof, or the pharmaceutically acceptable salt thereof according to claim 1 or 2, wherein:
W is selected from the group consisting of C and N, wherein when W is N, R¹ is absent;
X is selected from the group consisting of hydrogen and fluorine; Y is selected from the group consisting of hydrogen and fluorine; Z is selected from the group consisting of hydrogen and fluorine; and at least two of X, Y and Z are not hydrogen;
T₁ and T₂ are selected from the group consisting of C and N, wherein when T₁ is N, R^{2c} is absent, when T₂ is N, R^{2b} is absent, and T₁ and T₂ are not both N;
R¹ is selected from the group consisting of chlorine, trifluoromethyl, and difluoromethyl; R^{2a}, R^{2b}, and R^{2c} are each independently selected from the group consisting of hydrogen, methoxy, fluorine, and chlorine; R³ is methyl; R⁴ is selected from the group consisting of hydrogen and fluorine; and R⁵ is selected from the group consisting of methyl and difluoromethyl.

8. The compound, or the isomer thereof, or the racemate thereof, or the pharmaceutically acceptable salt thereof according to claim 1 or 2, wherein the compound is selected from the group consisting of:
| No. | Compound Structure | No. | Compound Structure |
|---|---|---|---|
| 1 | 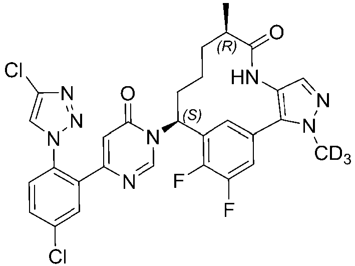 | 2 | 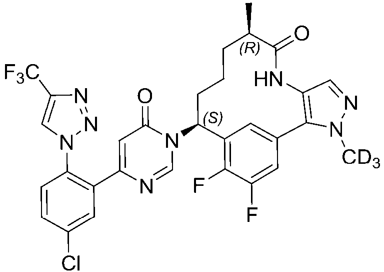 |
| 3 | 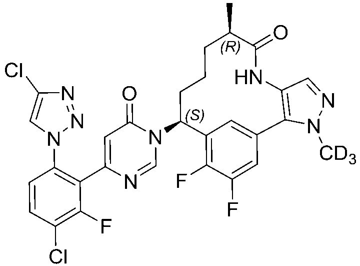 | 4 | 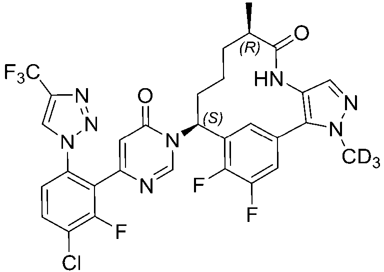 |
| 5 | 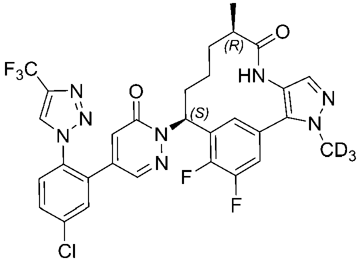 | 6 | 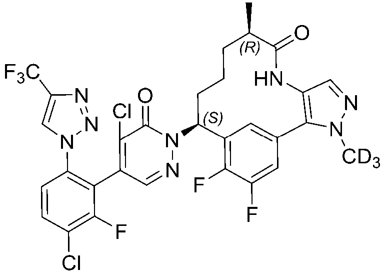 |
| 7 | 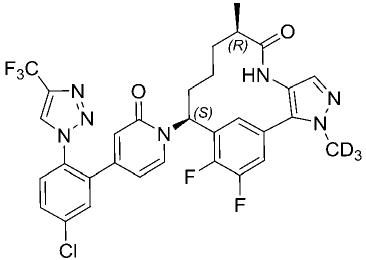 | 8 | 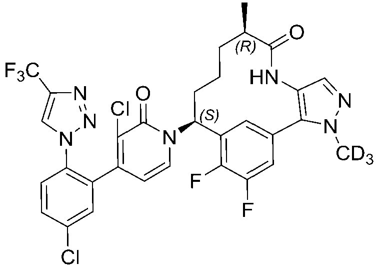 |
| 9 | 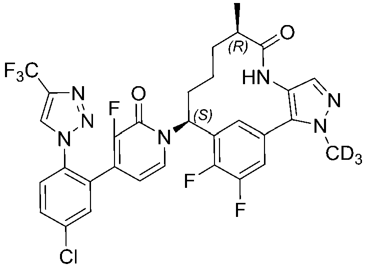 | 10 | 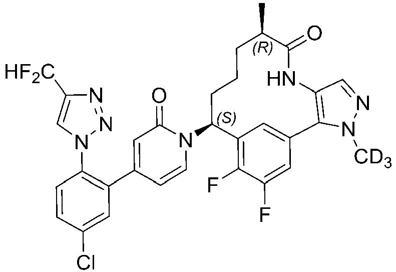 |
| 11 | 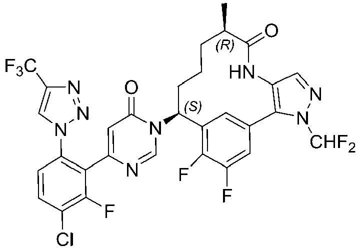 | 12 | 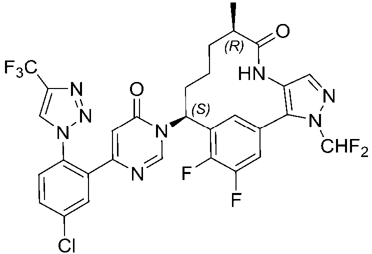 |
| 13 | 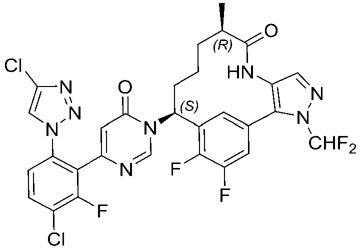 | 14 | 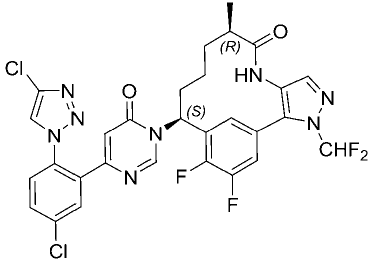 |
| 15 | 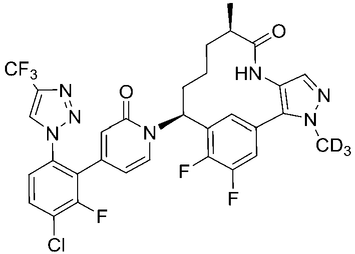 | 16 | 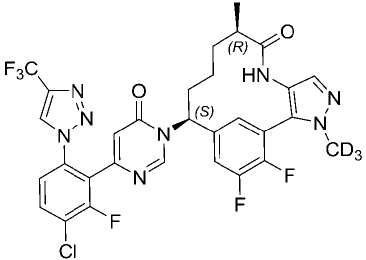 |
| 17 | 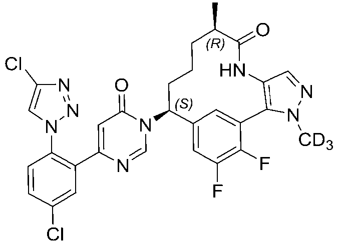 | 18 | 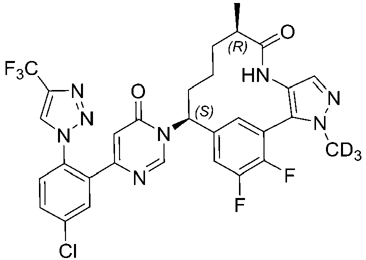 |
| 19 | 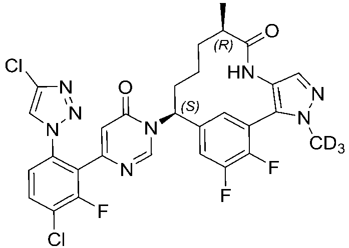 | 20 | 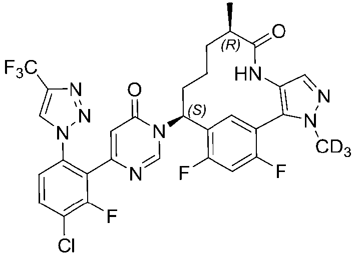 |
| 21 | 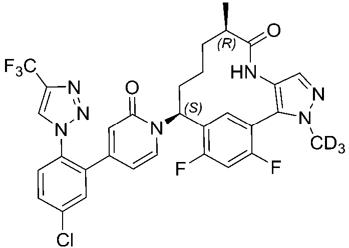 | 22 | 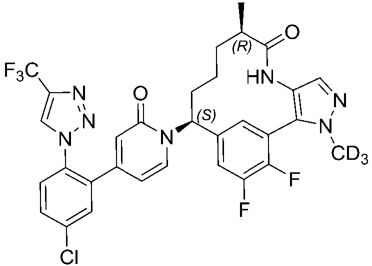 |
| 23 . | 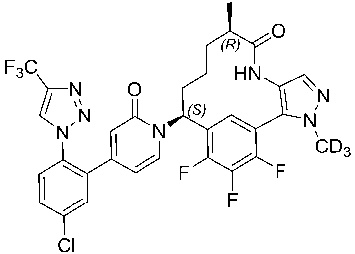 | | |

9. A pharmaceutical composition, comprising a therapeutically effective amount of the compound, or the isomer thereof, or the racemate thereof, or the pharmaceutically acceptable salt thereof according to any one of claims 1 to 8, and a pharmaceutically acceptable carrier.

10. Use of the compound, or the isomer thereof, or the racemate thereof, or the pharmaceutically acceptable salt thereof according to any one of claims 1 to 8 in the manufacture of a medicament for treating an FXIa-related disease, preferably a thrombus-related disease.
